(19) Europäisches Patentamt
European Patent Office
Office européen des brevets

(11) Publication number: **0 481 604 A1**

(12) **EUROPEAN PATENT APPLICATION**

(21) Application number: **91308508.0**

(51) Int. Cl.[5]: **C07D 239/36**, A01N 43/54

(22) Date of filing: **18.09.91**

(30) Priority: **16.10.90 GB 9022445**

(43) Date of publication of application: **22.04.92 Bulletin 92/17**

(84) Designated Contracting States: **CH DE ES FR GB GR IT LI NL**

(71) Applicant: **IMPERIAL CHEMICAL INDUSTRIES PLC**
**Imperial Chemical House, Millbank**
**London SW1P 3JF(GB)**

(72) Inventor: **Salmon, Roger**
**38 Tawfield**
**Bracknell, Berkshire(GB)**
Inventor: **Sunley, Raymond Leo**
**1 Mallard Close**
**Twyford, Berkshire RG10 0BE(GB)**
Inventor: **Whittle, Alan John**
**30 Kibblewhite Crescent**
**Twyford, Berkshire RG10 9AX(GB)**

(74) Representative: **Ricks, Michael James et al**
**Imperial Chemical Industries PLC Legal Department: Patents PO Box 6 Bessemer Road**
**Welwyn Garden City Herts, AL7 1HD(GB)**

(54) **Heterocyclic compounds.**

(57) Compound of formula (I) have insecticidal activity

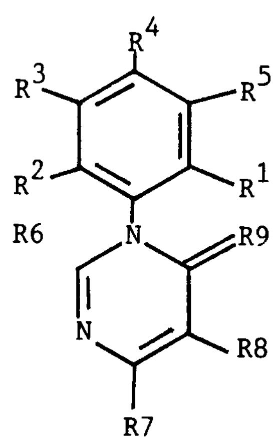

(I)

wherein $R^1$ is cyano or is an optionally substituted amide or thioamide group; $R^2$, $R^3$, $R^4$, $R^5$, $R^6$ and $R^7$ are defined substituents on the phenyl and heterocycic ring respectively; $R^9$ is O or S; and $R^8$ is an optionally substituted alkene, alkyne, cyclopropyl, epoxy or carboxy alkyl group.

EP 0 481 604 A1

The present invention relates to novel phenyl substituted heterocyclic compounds which have insecticidal activity, to processes for their preparation and to their use as insecticides.

According to the present invention there is provided an insecticidal compound of formula (I), wherein $R^1$ is cyano or a group $-CX-NR^{10}R^{11}$ wherein X is O or S or S=O; and $R^{10}$ and $R^{11}$ are independently selected from hydrogen, nitro, amino or alkyl optionally substituted by halogen, by cycloalkyl, by formyl, by $C_{2-7}$ alkanoyl, by $C_{4-7}$ cycloalkylcarbonyl, by $C_{2-7}$ alkoxycarbonyl, by $C_{2-7}$ haloalkoxycarbonyl, by an aryl group or by an aromatic heterocyclic group or

$R^{10}$ and $R^{11}$ together with the nitrogen to which they are attached form an aliphatic heterocyclic group containing from 4 to 8 atoms in the ring and optionally substituted by halogen or alkyl or

$R^{10}$ and $R^{11}$ together form the group $=CHR^{12}$ wherein $R^{12}$ is alkyl, $C_{2-6}$ alkenyl, aryl, an aromatic heterocycle, or amino optionally substituted by alkyl or

$R^{10}$ is hydrogen and $R^{11}$ is alkoxycarbonyl, alkylcarbonyl, optionally substituted aralkyl or a group $-S(O)_nR^{13}$ where $R^{13}$ is alkyl, haloalkyl or cycloalkyl and n is 0, 1 or 2;

$R^2$ is hydrogen, halogen, haloalkyl, nitro, cyano, a group $-CX-NR^{10}R^{11}$ as hereinbefore defined, or

$R^2$ is $-C{\equiv}C-Y^1$ wherein $Y^1$ is hydrogen, an optionally halo-substituted alkyl group, an optionally halo-substituted alkoxy group, trialkylsilyl or $-COOY^2$ where $Y^2$ is alkyl, or $Y^1$ is $-CONY^3Y^4$ wherein $Y^3$ and $Y^4$ are independently selected from hydrogen and alkyl,

or $R^2$ is alkyl or alkoxy,

or $R^2$ is $S(O)_nR^{13}$ where $R^{13}$ and n are as previously defined,

or $R^2$ is $NY^5Y^6$ where $Y^5$ and $Y^6$ are independently selected from hydrogen and alkyl;

$R^3$ and $R^5$ are independently selected from hydrogen, halogen, alkyl, haloalkyl, cycloalkyl, nitro, $NY^7Y^6$ wherein $Y^7$ and $Y^6$ are independently selected from hydrogen and alkyl, $-C{\equiv}C-Y^1$, wherein $Y^1$ is as previously defined or $S(O)_nR^{13}$ where $R^{13}$ and n are as previously defined;

$R^4$ is halogen, haloalkyl, haloalkoxy or $S(O)_nR^{13}$ where $R^{13}$ and n are as previously defined;

$R^9$ is oxygen or sulphur;

$R^6$ is hydrogen, halogen, $NR^{14}R^{15}$, $S(O)_nR^{13}$, alkyl or cycloalkyl wherein $R^{14}$ and $R^{15}$ are independently selected from hydrogen, alkyl or cycloalkyl and $R^{13}$ and n are as defined previously;

$R^7$ is halo, nitro, haloalkyl, haloalkoxy or $S(O)_nR^{13}$ where $R^{13}$ and n are as defined previously; and

$R^8$ is $-CR^{16}=CR^{17}R^{18}$ wherein $R^{16}$, $R^{17}$, and $R^{18}$ are independently selected from hydrogen, from alkyl, from halogen, from haloalkyl, from alkoxy, from haloalkoxy, from haloalkylthio, from $COOR^{19}$ where $R^{19}$ is alkyl, from $NR^{20}R^{21}$ where $R^{20}$ and $R^{21}$ are independently selected from hydrogen, alkyl, haloalkyl and cycloalkyl, and from $S(O)_nR^{13}$ where $R^{13}$ and n are as previously defined,

or $R^8$ is $-C{\equiv}C-Y^1$ where $Y^1$ is as previously defined,

or $R^8$ is a group of formula (Ia), where A is $CH_2$ or CH-halogen or $C(halogen)_2$ or A is O and $R^{22}$, $R^{23}$ and $R^{24}$ are independently selected from hydrogen, alkyl, halogen, or haloalkyl,

or $R^8$ is a group of formula (Ib), where $R^{25}$ is alkyl or haloalkyl.

The term "alkyl" is used herein includes straight or branched chain alkyl groups, preferably containing up to 6 carbon atoms, for example from 1 to 4 carbon atoms. This applies also to alkyl moieties contained in groups such as "haloalkyl", "alkoxy" and "haloalkoxy". The term "cycloalkyl" used herein refers to a carbocyclic ring suitably having from 3 to 10 and preferably from 3 to 7 carbon atoms in the ring. The cycloalkyl group is preferably cyclopropyl.

Suitable halogen groups, $R^2$, $R^3$, $R^4$, $R^5$, $R^6$ and $R^7$ include fluorine, chlorine, bromine or iodine.

Suitable haloalkyl groups for $R^2$, $R^4$, $R^7$ and $R^{13}$ are $C_1$-$C_4$ alkyl groups substituted with chlorine, fluorine, bromine or iodine. Such groups may include di- and trihalomethyl groups in particular trifluoromethyl, and pentahaloethyl groups, in particular pentafluoroethyl. Such groups may also include two or more different halogens.

Suitable haloalkoxy groups for $R^4$ and $R^7$ include $C_1$-$C_4$ haloalkoxy groups, substituted with fluorine, chlorine, bromine, or iodine. Such groups may also include two or more different halogens.

Preferably $R^{10}$, $R^{11}$ are hydrogen or alkyl and X is O or S. It is especially preferred that $R^{10}$ and $R^{11}$ are hydrogen and X is O or S.

Preferably $R^3$ and $R^5$ are independently selected from hydrogen, halogen, alkyl, haloalkyl, or cycloalkyl;

Thus according to a further aspect of the present invention there is provided a compound of formula (I'), wherein $R^1$ is cyano or a group $-CX-NR^{10}R^{11}$ wherein X is O or S and $R^{10}$ and $R^{11}$ are independently selected from hydrogen or alkyl;

$R^2$ is hydrogen, halogen, haloalkyl, nitro, cyano or a group $-CX-NR^{10}R^{11}$ as hereinbefore defined;

$R^3$ and $R^5$ are independently selected from hydrogen, halogen, alkyl, haloalkyl, or cycloalkyl;

$R^4$ is halogen, haloalkyl, haloalkoxy or $S(O)_nR^{13}$ where $R^{13}$ and n are as previously defined;

$R^6$ is hydrogen, halogen, $NR^{14}R^{15}$, $S(O)_nR^{13}$, alkyl or cycloalkyl wherein $R^{14}$ and $R^{15}$ are independently

selected from hydrogen, alkyl or cycloalkyl and $R^{13}$ and n are as defined previously;
$R^7$ is halo, nitro, haloalkyl, haloalkoxy or $S(O)_nR^{13}$ where $R^{13}$ and n are as defined previously;
$R^8$ is $-CR^{16}=CR^{17}R^{18}$ wherein $R^{16}$, $R^{17}$, and $R^{18}$ are independently selected from hydrogen, from alkyl, from halogen, from haloalkyl, from alkoxy, from haloalkoxy, from haloalkylthio, from $COOR^{19}$ where $R^{19}$ is alkyl, from $NR^{20}R^{21}$ where $R^{20}$ and $R^{21}$ are independently selected from hydrogen, alkyl, haloalkyl and cycloalkyl, and from $S(O)_nR^{13}$ where $R^{13}$ and n are as previously defined,
or $R^8$ is $-C\equiv C-Y^1$ where $Y^1$ is hydrogen, alkyl or haloalkyl,
or $R^8$ is a group of formula (Ia), where A is $CH_2$ or CH(halogen) or $C(halogen)^2$ or A is O and $R^{22}$, $R^{23}$ and $R^{24}$ are independently selected from hydrogen, alkyl, halogen, or haloalkyl,
or $R^8$ is a group of formula (Ib), where $R^{25}$ is alkyl or haloalkyl; and
$R^9$ is oxygen or sulphur.

Preferably $R^4$ is trifluoromethyl, pentafluoroethyl trifluoromethylthio, iodine, bromine, chlorine, trifluoromethoxy or methylthio or $-S(O)_nR^{13}$, for example $-SOCF_3$. It is especially preferred that $R^4$ is trifluoromethyl.

Preferably $R^3$ and $R^5$ are hydrogen.

Preferably $R^2$ is fluorine, chlorine, bromine, cyano, or trifluoromethyl.

Preferably $R^1$ is cyano.

Preferably $R^6$ is hydrogen.

Preferably $R^7$ is trifluoromethyl or pentafluoroethyl.

In the group $R^8$, it is preferred that $R^{16}$, $R^{17}$ and $R^{18}$ are independently selected from hydrogen, methyl, ethyl, methoxy and ethoxy; preferably $Y^1$ is hydrogen or alkyl; preferably $R^{22}$, $R^{23}$, and $R^{24}$ are independently selected from hydrogen, methyl or ethyl; and preferably $R^{25}$ is alkyl, for example methyl or ethyl.

Preferably $R^9$ is oxygen.

Examples of compounds of formula (I') wherein $R^3$, $R^5$ and $R^6$ are hydrogen are set out in Table I below.

TABLE I

| COMPOUND NO. | $R^1$ | $R^2$ | $R^4$ | $R^7$ | $R^8$ | $R^9$ |
|---|---|---|---|---|---|---|
| 1 | CN | Cl | $CF_3$ | $CF_3$ | $-CH=CH_2$ | O |
| 2 | CN | Cl | $CF_3$ | $CF_3$ | $-C(OC_2H_5)=CH_2$ | O |
| 3 | CN | Cl | $CF_3$ | $CF_3$ | $-CO-CH_3$ | O |
| 4 | CN | Cl | $CF_3$ | $CF_3$ | $-C(OCH_3)=CH_2$ | O |
| 5 | CN | Cl | $CF_3$ | $CF_3$ | $-C(CH_3)=CH_2$ | O |
| 6 | $CONH_2$ | Cl | $CF_3$ | $CF_3$ | $-C(CH_3)=CH_2$ | O |
| 7 | $CSNH_2$ | Cl | $CF_3$ | $CF_3$ | $-C(CH_3)=CH_2$ | O |
| 8 | $CON(CH_3)_2$ | Cl | $CF_3$ | $CF_3$ | $-C(CH_3)=CH_2$ | O |
| 9 | $CSN(CH_3)_2$ | Cl | $CF_3$ | $CF_3$ | $-C(CH_3)=CH_2$ | O |
| 10 | CN | Cl | $CF_3$ | $CF_3$ | -C - $CH_2$<br>/ \ /<br>$CH_3$ O | O |
| 11 | CN | Cl | $CF_3$ | $CF_3$ | $-C\equiv CH$ | O |
| 12 | CN | Cl | $CF_3$ | $CF_3$ | $-C\equiv C-CH_3$ | O |
| 13 | CN | H | $CF_3$ | $CF_3$ | $-CH=CH_2$ | O |
| 14 | CN | H | $CF_3$ | $CF_3$ | $-C(OC_2H_5)=CH_2$ | O |
| 15 | CN | H | $CF_3$ | $CF_3$ | $-CO-CH_3$ | O |
| 16 | CN | H | $CF_3$ | $CF_3$ | $-C(OCH_3)=CH_2$ | O |
| 17 | CN | H | $CF_3$ | $CF_3$ | $-C(CH_3)=CH_2$ | O |
| 18 | $CONH_2$ | H | $CF_3$ | $CF_3$ | $-C(CH_3)=CH_2$ | O |
| 19 | $CSNH_2$ | H | $CF_3$ | $CF_3$ | $-C(CH_3)=CH_2$ | O |
| 20 | $CON(CH_3)_2$ | H | $CF_3$ | $CF_3$ | $-C(CH_3)=CH_2$ | O |
| 21 | $CSN(CH_3)_2$ | H | $CF_3$ | $CF_3$ | $-C(CH_3)=CH_2$ | O |

TABLE I (continued)

| COMPOUND NO. | $R^1$ | $R^2$ | $R^4$ | $R^7$ | $R^8$ | $R^9$ |
|---|---|---|---|---|---|---|
| 22 | CN | H | $CF_3$ | $CF_3$ | $-C - CH_2$<br>/ \ /<br>$CH_3$ O | 0 |
| 23 | CN | H | $CF_3$ | $CF_3$ | $-C{\equiv}CH$ | 0 |
| 24 | CN | H | $CF_3$ | $CF_3$ | $-C{\equiv}C-CH_3$ | 0 |
| 25 | CN | H | $CF_3$ | $C_2F_5$ | $-CH=CH_2$ | 0 |
| 26 | CN | H | $CF_3$ | $C_2F_5$ | $-C(OC_2H_5)=CH_2$ | 0 |
| 27 | CN | H | $CF_3$ | $C_2F_5$ | $-CO-CH_3$ | 0 |
| 28 | CN | H | $CF_3$ | $C_2F_5$ | $-C(OCH_3)=CH_2$ | 0 |
| 29 | CN | H | $CF_3$ | $C_2F_5$ | $-C(CH_3)=CH_2$ | 0 |
| 30 | $CONH_2$ | H | $CF_3$ | $C_2F_5$ | $-C(CH_3)=CH_2$ | 0 |
| 31 | $CSNH_2$ | H | $CF_3$ | $C_2F_5$ | $-C(CH_3)=CH_2$ | 0 |
| 32 | $CON(CH_3)_2$ | H | $CF_3$ | $C_2F_5$ | $-C(CH_3)=CH_2$ | 0 |
| 33 | $CSN(CH_3)_2$ | H | $CF_3$ | $C_2F_5$ | $-C(CH_3)=CH_2$ | 0 |
| 34 | CN | H | $CF_3$ | $C_2F_5$ | $-C - CH_2$<br>/ \ /<br>$CH_3$ O | 0 |
| 35 | CN | H | $CF_3$ | $C_2F_5$ | $-C{\equiv}CH$ | 0 |
| 36 | CN | H | $CF_3$ | $C_2F_5$ | $-C{\equiv}C-CH_3$ | 0 |
| 37 | CN | Cl | $CF_3$ | $C_2F_5$ | $-CH=CH_2$ | 0 |
| 38 | CN | Cl | $CF_3$ | $C_2F_5$ | $-C(OC_2H_5)=CH_2$ | 0 |
| 39 | CN | Cl | $CF_3$ | $C_2F_5$ | $-CO-CH_3$ | 0 |
| 40 | CN | Cl | $CF_3$ | $C_2F_5$ | $-C(OCH_3)=CH_2$ | 0 |
| 41 | CN | Cl | $CF_3$ | $C_2F_5$ | $-C(CH_3)=CH_2$ | 0 |
| 42 | $CONH_2$ | Cl | $CF_3$ | $C_2F_5$ | $-C(CH_3)=CH_2$ | 0 |
| 43 | $CSNH_2$ | Cl | $CF_3$ | $C_2F_5$ | $-C(CH_3)=CH_2$ | 0 |
| 44 | $CON(CH_3)_2$ | Cl | $CF_3$ | $C_2F_5$ | $-C(CH_3)=CH_2$ | 0 |
| 45 | $CSN(CH_3)_2$ | Cl | $CF_3$ | $C_2F_5$ | $-C(CH_3)=CH_2$ | 0 |
| 46 | CN | Cl | $CF_3$ | $C_2F_5$ | $-C - CH_2$<br>/ \ /<br>$CH_3$ O | 0 |

TABLE I (continued)

| COMPOUND NO. | $R^1$ | $R^2$ | $R^4$ | $R^7$ | $R^8$ | $R^9$ |
|---|---|---|---|---|---|---|
| 47 | CN | Cl | $CF_3$ | $C_2F_5$ | $-C\equiv CH$ | 0 |
| 48 | CN | Cl | $CF_3$ | $C_2F_5$ | $-C\equiv C-CH_3$ | 0 |
| 49 | CN | Br | $CF_3$ | $CF_3$ | $-CH=CH_2$ | 0 |
| 50 | CN | Br | $CF_3$ | $CF_3$ | $-C(OC_2H_5)=CH_2$ | 0 |
| 51 | CN | Br | $CF_3$ | $CF_3$ | $-CO-CH_3$ | 0 |
| 52 | CN | Br | $CF_3$ | $CF_3$ | $-C(OCH_3)=CH_2$ | 0 |
| 53 | CN | Br | $CF_3$ | $CF_3$ | $-C(CH_3)=CH_2$ | 0 |
| 54 | $CONH_2$ | Br | $CF_3$ | $CF_3$ | $-C(CH_3)=CH_2$ | 0 |
| 55 | $CSNH_2$ | Br | $CF_3$ | $CF_3$ | $-C(CH_3)=CH_2$ | 0 |
| 56 | $CON(CH_3)_2$ | Br | $CF_3$ | $CF_3$ | $-C(CH_3)=CH_2$ | 0 |
| 57 | $CSN(CH_3)_2$ | Br | $CF_3$ | $CF_3$ | $-C(CH_3)=CH_2$ | 0 |
| 58 | CN | Br | $CF_3$ | $CF_3$ | -C - $CH_2$<br>/ \ /<br>$CH_3$ O | 0 |
| 59 | CN | Br | $CF_3$ | $CF_3$ | $-C\equiv CH$ | 0 |
| 60 | CN | Br | $CF_3$ | $CF_3$ | $-C\equiv C-CH_3$ | 0 |
| 61 | CN | Br | $CF_3$ | $C_2F_5$ | $-CH=CH_2$ | 0 |
| 62 | CN | Br | $CF_3$ | $C_2F_5$ | $-C(OC_2H_5)=CH_2$ | 0 |
| 63 | CN | Br | $CF_3$ | $C_2F_5$ | $-CO-CH_3$ | 0 |
| 64 | CN | Br | $CF_3$ | $C_2F_5$ | $-C(OCH_3)=CH_2$ | 0 |
| 65 | CN | Br | $CF_3$ | $C_2F_5$ | $-C(CH_3)=CH_2$ | 0 |
| 66 | $CONH_2$ | Br | $CF_3$ | $C_2F_5$ | $-C(CH_3)=CH_2$ | 0 |
| 67 | $CSNH_2$ | Br | $CF_3$ | $C_2F_5$ | $-C(CH_3)=CH_2$ | 0 |
| 68 | $CON(CH_3)_2$ | Br | $CF_3$ | $C_2F_5$ | $-C(CH_3)=CH_2$ | 0 |
| 69 | $CSN(CH_3)_2$ | Br | $CF_3$ | $C_2F_5$ | $-C(CH_3)=CH_2$ | 0 |
| 70 | CN | Br | $CF_3$ | $C_2F_5$ | -C - $CH_2$<br>/ \ /<br>$CH_3$ O | 0 |
| 71 | CN | Br | $CF_3$ | $C_2F_5$ | $-C\equiv CH$ | 0 |
| 72 | CN | Br | $CF_3$ | $C_2F_5$ | $-C\equiv C-CH_3$ | 0 |
| 73 | CN | $NO_2$ | $CF_3$ | $CF_3$ | $-CH=CH_2$ | 0 |

TABLE I (continued)

| COMPOUND NO. | $R^1$ | $R^2$ | $R^4$ | $R^7$ | $R^8$ | $R^9$ |
|---|---|---|---|---|---|---|
| 74 | CN | $NO_2$ | $CF_3$ | $CF_3$ | $-C(OC_2H_5)=CH_2$ | 0 |
| 75 | CN | $NO_2$ | $CF_3$ | $CF_3$ | $-CO-CH_3$ | 0 |
| 76 | CN | $NO_2$ | $CF_3$ | $CF_3$ | $-C(OCH_3)=CH_2$ | 0 |
| 77 | CN | $NO_2$ | $CF_3$ | $CF_3$ | $-C(CH_3)=CH_2$ | 0 |
| 78 | $CONH_2$ | $NO_2$ | $CF_3$ | $CF_3$ | $-C(CH_3)=CH_2$ | 0 |
| 79 | $CSNH_2$ | $NO_2$ | $CF_3$ | $CF_3$ | $-C(CH_3)=CH_2$ | 0 |
| 80 | $CON(CH_3)_2$ | $NO_2$ | $CF_3$ | $CF_3$ | $-C(CH_3)=CH_2$ | 0 |
| 81 | $CSN(CH_3)_2$ | $NO_2$ | $CF_3$ | $CF_3$ | $-C(CH_3)=CH_2$ | 0 |
| 82 | CN | $NO_2$ | $CF_3$ | $CF_3$ | -C - $CH_2$<br>/ \ /<br>$CH_3$ O | 0 |
| 83 | CN | $NO_2$ | $CF_3$ | $CF_3$ | -C≡CH | 0 |
| 84 | CN | $NO_2$ | $CF_3$ | $CF_3$ | $-C≡C-CH_3$ | 0 |
| 85 | CN | Cl | $CF_3$ | nC3F7 | $-CH=CH_2$ | 0 |
| 86 | CN | Cl | $CF_3$ | nC3F7 | $-C(OC_2H_5)=CH_2$ | 0 |
| 87 | CN | Cl | $CF_3$ | nC3F7 | $-CO-CH_3$ | 0 |
| 88 | CN | Cl | $CF_3$ | nC3F7 | $-C(OCH_3)=CH_2$ | 0 |
| 89 | CN | Cl | $CF_3$ | nC3F7 | $-C(CH_3)=CH_2$ | 0 |
| 90 | $CONH_2$ | Cl | $CF_3$ | nC3F7 | $-C(CH_3)=CH_2$ | 0 |
| 91 | $CSNH_2$ | Cl | $CF_3$ | nC3F7 | $-C(CH_3)=CH_2$ | 0 |
| 92 | $CON(CH_3)_2$ | Cl | $CF_3$ | nC3F7 | $-C(CH_3)=CH_2$ | 0 |
| 93 | $CSN(CH_3)_2$ | Cl | $CF_3$ | nC3F7 | $-C(CH_3)=CH_2$ | 0 |
| 94 | CN | Cl | $CF_3$ | nC3F7 | -C - $CH_2$<br>/ \ /<br>$CH_3$ O | 0 |
| 95 | CN | Cl | $CF_3$ | nC3F7 | -C≡CH | 0 |
| 96 | CN | Cl | $CF_3$ | nC3F7 | $-C≡C-CH_3$ | 0 |
| 97 | CN | CN | $CF_3$ | $CF_3$ | $-CH=CH_2$ | 0 |
| 98 | CN | CN | $CF_3$ | $CF_3$ | $-C(OC_2H_5)=CH_2$ | 0 |
| 99 | CN | CN | $CF_3$ | $CF_3$ | $-CO-CH_3$ | 0 |
| 100 | CN | CN | $CF_3$ | $CF_3$ | $-C(OCH_3)=CH_2$ | 0 |
| 101 | CN | CN | $CF_3$ | $CF_3$ | $-C(CH_3)=CH_2$ | 0 |

TABLE I (continued)

| COMPOUND NO. | $R^1$ | $R^2$ | $R^4$ | $R^7$ | $R^8$ | $R^9$ |
|---|---|---|---|---|---|---|
| 102 | $CONH_2$ | CN | $CF_3$ | $CF_3$ | $-C(CH_3)=CH_2$ | 0 |
| 103 | $CSNH_2$ | CN | $CF_3$ | $CF_3$ | $-C(CH_3)=CH_2$ | 0 |
| 104 | $CON(CH_3)_2$ | CN | $CF_3$ | $CF_3$ | $-C(CH_3)=CH_2$ | 0 |
| 105 | $CSN(CH_3)_2$ | CN | $CF_3$ | $CF_3$ | $-C(CH_3)=CH_2$ | 0 |
| 106 | CN | CN | $CF_3$ | $CF_3$ | $-C - CH_2$ <br> / \ / <br> $CH_3$ O | 0 |
| 107 | CN | CN | $CF_3$ | $CF_3$ | $-C\equiv CH$ | 0 |
| 108 | CN | CN | $CF_3$ | $CF_3$ | $-C\equiv C-CH_3$ | 0 |
| 109 | CN | CN | $CF_3$ | $C_2F_5$ | $-CH=CH_2$ | 0 |
| 110 | CN | CN | $CF_3$ | $C_2F_5$ | $-C(OC_2H_5)=CH_2$ | 0 |
| 111 | CN | CN | $CF_3$ | $C_2F_5$ | $-CO-CH_3$ | 0 |
| 112 | CN | CN | $CF_3$ | $C_2F_5$ | $-C(OCH_3)=CH_2$ | 0 |
| 113 | CN | CN | $CF_3$ | $C_2F_5$ | $-C(CH_3)=CH_2$ | 0 |
| 114 | $CONH_2$ | CN | $CF_3$ | $C_2F_5$ | $-C(CH_3)=CH_2$ | 0 |
| 115 | $CSNH_2$ | CN | $CF_3$ | $C_2F_5$ | $-C(CH_3)=CH_2$ | 0 |
| 116 | $CON(CH_3)_2$ | CN | $CF_3$ | $C_2F_5$ | $-C(CH_3)=CH_2$ | 0 |
| 117 | $CSN(CH_3)_2$ | CN | $CF_3$ | $C_2F_5$ | $-C(CH_3)=CH_2$ | 0 |
| 118 | CN | CN | $CF_3$ | $C_2F_5$ | $-C - CH_2$ <br> / \ / <br> $CH_3$ O | 0 |
| 119 | CN | CN | $CF_3$ | $C_2F_5$ | $-C\equiv CH$ | 0 |
| 120 | CN | CN | $CF_3$ | $C_2F_5$ | $-C\equiv C-CH_3$ | 0 |
| 121 | CN | F | $CF_3$ | $CF_3$ | $-CH=CH_2$ | 0 |
| 122 | CN | F | $CF_3$ | $CF_3$ | $-C(OC_2H_5)=CH_2$ | 0 |
| 123 | CN | F | $CF_3$ | $CF_3$ | $-CO-CH_3$ | 0 |
| 124 | CN | F | $CF_3$ | $CF_3$ | $-C(OCH_3)=CH_2$ | 0 |
| 125 | CN | F | $CF_3$ | $CF_3$ | $-C(CH_3)=CH_2$ | 0 |
| 126 | $CONH_2$ | F | $CF_3$ | $CF_3$ | $-C(CH_3)=CH_2$ | 0 |
| 127 | $CSNH_2$ | F | $CF_3$ | $CF_3$ | $-C(CH_3)=CH_2$ | 0 |
| 128 | $CON(CH_3)_2$ | F | $CF_3$ | $CF_3$ | $-C(CH_3)=CH_2$ | 0 |
| 129 | $CSN(CH_3)_2$ | F | $CF_3$ | $CF_3$ | $-C(CH_3)=CH_2$ | 0 |

TABLE I (continued)

| COMPOUND NO. | $R^1$ | $R^2$ | $R^4$ | $R^7$ | $R^8$ | $R^9$ |
|---|---|---|---|---|---|---|
| 130 | CN | F | $CF_3$ | $CF_3$ | -C - $CH_2$ / \ / $CH_3$ O | O |
| 131 | CN | F | $CF_3$ | $CF_3$ | -C≡CH | O |
| 132 | CN | F | $CF_3$ | $CF_3$ | $-C≡C-CH_3$ | O |
| 133 | CN | F | $CF_3$ | $C_2F_5$ | $-CH=CH_2$ | O |
| 134 | CN | F | $CF_3$ | $C_2F_5$ | $-C(OC_2H_5)=CH_2$ | O |
| 135 | CN | F | $CF_3$ | $C_2F_5$ | $-CO-CH_3$ | O |
| 136 | CN | F | $CF_3$ | $C_2F_5$ | $-C(OCH_3)=CH_2$ | O |
| 137 | CN | F | $CF_3$ | $C_2F_5$ | $-C(CH_3)=CH_2$ | O |
| 138 | $CONH_2$ | F | $CF_3$ | $C_2F_5$ | $-C(CH_3)=CH_2$ | O |
| 139 | $CSNH_2$ | F | $CF_3$ | $C_2F_5$ | $-C(CH_3)=CH_2$ | O |
| 140 | $CON(CH_3)_2$ | F | $CF_3$ | $C_2F_5$ | $-C(CH_3)=CH_2$ | O |
| 141 | $CSN(CH_3)_2$ | F | $CF_3$ | $C_2F_5$ | $-C(CH_3)=CH_2$ | O |
| 142 | CN | F | $CF_3$ | $C_2F_5$ | -C - $CH_2$ / \ / $CH_3$ O | O |
| 143 | CN | F | $CF_3$ | $C_2F_5$ | -C≡CH | O |
| 144 | CN | F | $CF_3$ | $C_2F_5$ | $-C≡C-CH_3$ | O |
| 145 | CN | Cl | $OCF_3$ | $C_2F_5$ | $-CH=CH_2$ | O |
| 146 | CN | Cl | $OCF_3$ | $C_2F_5$ | $-C(OC_2H_5)=CH_2$ | O |
| 147 | CN | Cl | $OCF_3$ | $C_2F_5$ | $-CO-CH_3$ | O |
| 148 | CN | Cl | $OCF_3$ | $C_2F_5$ | $-C(OCH_3)=CH_2$ | O |
| 149 | CN | Cl | $OCF_3$ | $C_2F_5$ | $-C(CH_3)=CH_2$ | O |
| 150 | $CONH_2$ | Cl | $OCF_3$ | $C_2F_5$ | $-C(CH_3)=CH_2$ | O |
| 151 | $CSNH_2$ | Cl | $OCF_3$ | $C_2F_5$ | $-C(CH_3)=CH_2$ | O |
| 152 | $CON(CH_3)_2$ | Cl | $OCF_3$ | $C_2F_5$ | $-C(CH_3)=CH_2$ | O |
| 153 | $CSN(CH_3)_2$ | Cl | $OCF_3$ | $C_2F_5$ | $-C(CH_3)=CH_2$ | O |
| 154 | CN | Cl | $OCF_3$ | $C_2F_5$ | -C - $CH_2$ / \ / $CH_3$ O | O |
| 155 | CN | Cl | $OCF_3$ | $C_2F_5$ | -C≡CH | O |
| 156 | CN | Cl | $OCF_3$ | $C_2F_5$ | $-C≡C-CH_3$ | O |

Also to be considered as specifically disclosed as Compound Nos 157 to 318 are compounds corresponding exactly to Compound Nos 1 to 156 above wherein $R^9$ is S in place of O.

Compounds of formula (I) can be prepared by reacting a compound of formula (II), wherein $R^1$, $R^2$, $R^3$, $R^4$ and $R^5$ are as defined in relation to formula (I) and $R^{26}$ is a leaving group; with a compound of formula (III), wherein $R^6$, $R^7$, $R^8$ and $R^9$ are as defined in relation to formula (I) and thereafter if desired (a)

converting a group $R^2$-$R^5$ to a different such group, and/or (b) converting a group $R^6$ to $R^9$ to a different such group.

The reaction is suitably carried out in the presence of a solvent and a base. The base may be for example an alkali metal hydride, an alkali metal alkoxide or an alkali metal carbonate, and the solvent may be a hydrocarbon solvent, such as petroleum ether, toluene, an alcohol, an ether, such as tetrahydrofuran, or an aprotic polar solvent such as dimethylformamide or dimethylacetamide.

The leaving group $R^{26}$ is preferably halogen.

If necessary an appropriate catalyst such as a crown ether, potassium fluoride or copper can be added depending upon the precise nature of $R^{26}$.

Preferably, however compounds of formula (I) can be prepared by replacement of a precursor group by the desired group $R^1$ to $R^9$. Examples of such replacement reactions will now be considered for the various relevant groups.

A cyano group, such as $R^1$, may be introduced by the replacement of a nitro group precursor which is first converted to an amino group.

Thus a compound of formula (I) may be prepared by the following stages:-

(a) preparation of a compound of formula (Ic), by reacting a compound of formula (IIa), wherein $R^1$, $R^2$, $R^3$, $R^4$, and $R^5$ are as defined in relation to formula (I) and $R^{26}$ is a leaving group as defined above, with a compound of formula (III); and

(b) reduction of the nitro group to form a compound of formula (Id), by reacting with a reducing agent, such as stannous chloride, in acid conditions, for example, in a solution in concentrated hydrochloric acid. Moderate temperatures of from 2 to 45°C are suitably employed. Alternatively, the reduction may be carried out using reduced iron powder in a hydroxylic solvent, such as isopropanol in the presence of an acid catalyst, for example, hydrochloric acid. Moderate temperatures of from 2 to 90°C are suitably employed; and either

(c) subsequent cyanation of the compound of formula (Id) by, for example, reaction with t-butylnitrite and copper cyanide. This step is suitably carried out in an organic solvent such as acetonitrile at low temperatures of from -20°C to +20°C preferably at about 0°C; or

(d) halogenation of the compound of formula (Id) by, for example, reaction with t-butylnitrite and a copper halide salt such as copper (II) iodide or copper (II) bromide. This step is suitably carried out in an organic solvent such as acetonitrile at low temperatures of from -20°C to +20°C preferably at about 0°C. The halide product is then further reacted with copper (I) cyanide in the presence of copper (I) iodide in a dipolar aprotic solvent such as dimethylformamide or N-methyl-pyrollidone at 150-180°C to give a compound of formula (I).

Compounds of formula (I) wherein $R^1$ is the group $-CX-NR^{10}R^{11}$ can be are prepared by conversion of the cyano group in a compound of formula (A) into the group $-CX-NR^{10}R^{11}$, wherein Pm is a group of formula (A').

Thus hydrolysis of the compound of formula (A), for example by treatment with concentrated aqueous acid optionally containing a small proportion of water, gives the corresponding compound of formula (I) wherein X is O and $R^{10}$ and $R^{11}$ are both hydrogen.

Treatment of the compound of formula (A) with a compound of formula alkyl-$CS-NH_2$, for example thioacetamide, gives the corresponding compound of formula (I) wherein X is S and $R^{10}$ and $R^{11}$ are hydrogen. The reaction suitably takes place in the presence of dry gaseous hydrochloric acid following the process described in J.A.C.S. (1960) 82 2656 (Taylor and Zoltewicz). A suitable solvent for the reaction is dimethylformamide.

Compounds of formula (I) wherein $R^{10}$ and $R^{11}$ are other than hydrogen may be conveniently prepared from the corresponding compound of formula (I) wherein both $R^{10}$ and $R^{11}$ are hydrogen. A suitable reaction scheme is shown below as Scheme 1.

The dehydrating agent used in step (b) above is suitably a carbodiimide dehydrating agent, for example dicyclohexylcarbodiimide or N-dimethylaminopropyl-N'-ethyl-carbodiimide (preferably as its salt with hydrochloric acid).

Compounds wherein X is S may also be prepared from the corresponding compound wherein X is O, for example by chlorination and subsequent treatment with hydrogen sulphide as shown in Scheme 2 when $R^{10}$ and $R^{11}$ are both alkyl. Alternatively, Lawesson's reagent may be used to convert a group X which is O into S.

The final product may be converted into a corresponding compound in which X is S=O by oxidation, for example using hydrogen peroxide or m-chloroperbenzoic acid.

When $R^{10}$ is hydrogen, a similar reaction may be used, but the product of step (a) is no longer the amine salt as shown in Scheme 2'.

The final product may be converted into a corresponding compound in which X is S=O by oxidation, for example using hydrogen peroxide or m-chloroperbenzoic acid.

The group $R^2$ may be introduced into compounds of formula (I) wherein $R^1$ is cyano by conversion of a precursor group $Z^2$ in a compound of formula (B) into the group $R^2$. The cyano group may if desired subsequently be converted into a group $-CX-NR^{10}R^{11}$ as described above, where $R^3$, $R^4$, $R^5$ and Pm are as defined previously.

The exact nature of the group $Z^2$ will depend on the conversion reaction envisaged. $Z^2$ may itself be a group $R^2$ which is a precursor for a different group $R^2$.

Examples of the conversion of a precursor group, $Z^2$, into a group $R^2$ are given in Schemes 3 and 4 below.

In scheme 3, X is a protective group such as trimethylsilyl and $Z^2$ is a leaving group such as halogen, for example bromo. Reaction (1) suitably takes place in the presence of a catalyst such as $Pd(II)Cl_2(Ph_3P)$ (wherein Ph is phenyl) or $Pd^\circ(PPh_3)_4$. Reaction (2) is a conventional reaction to remove the protective group, X, for example using halide ion such as fluoride in a suitable solvent such as tetrahydrofuran. Reaction (4) is an oxidation reaction which may be undertaken for example using potassium chromate or m-chloro-perbenzoic acid. The oxidation obtained (i.e. the value of the integer n as 1 or 2) will depend on the conditions used.

Scheme 4 illustrates preparations of compounds of formula (I) from the compound of formula (B) wherein the precursor, $Z^2$, is $NO_2$. The reduction of nitro to amino in reaction (5) conveniently takes place as described below. The methylation reaction (6) conveniently takes place under the action of dimethyl sulphate and base or under the action of formaldehyde in formic acid. Reaction 7 is a diazotisation procedure and takes place under the action of, for example t-butyl nitrite in the presence of dimethyl disulphide. Reaction (8) is an oxidation reaction which may take place for example under the same conditions as indicated for reaction (4) of scheme 3.

Similarly, Compounds of formula (I) may be prepared by conversion of a precursor group $Z^5$ in a compound of formula (C) into the group $R^5$. The exact nature of the group $Z^5$ will depend on the conversion reaction envisaged. $Z^5$ may itself be a group $R^5$ which is a precursor for a different group $R^5$.

It is preferred that $Z^5$ is an electrophilic group such as nitro or halogen, for example chloro, or bromo. A group $Z^5$ which is a halogen leaving group such as bromo may be converted into a group -C≡C-H by reaction of compound (C) with trimethylsilyl acetylene in the presence of a catalyst such as $Pd(II)Cl_2(Ph_3P)$ (wherein Ph is phenyl) or $Pd^\circ(PPh_3)_4$. The reaction is followed by removal of the protective trimethylsilyl group, for example using halide ion such as fluoride in a suitable solvent such as tetrahydrofuran (compare reactions 1 and 2 in scheme 3).

Similarly, a group $Z^5$ which is a halogen leaving group such as bromo may be converted into a group $-SCF_3$ by reaction with a suitable copper salt such as for example $CuSCF_3$.

A group $Z^5$ which is nitro may be reduced to amino as described below and may then be reacted with an alkylating agent to form a group $NY^5Y^6$ where one or both of the groups $Y^5$ and $Y^6$ is alkyl (compare reactions 5 and 6 of scheme 4). Alternatively, the amino group may be reacted with t-butyl nitrite in the presence of dimethyldisulphide to form a group $-SCH_3$ (compare reaction 7 of scheme 4).

A group $-SR^{13}$ may be oxidised to a group $-S(O)_nR^{13}$ using a suitable oxidising agent such as potassium chromate or m-chloro-perbenzoic acid (compare reaction 4 of scheme 3 and reaction 8 of scheme 4).

The compound of formula (C) above may be made by coupling a compound of formula (C II) with a compound of formula (III), wherein $R^2$, $R^3$, $R^4$, $R^{26}$ and $Z^5$ have the meanings given previously. The reaction takes place under the conditions described for the reaction of a compound of formula (II) and a compound of formula (III).

The compound of formula (C II) (for example when $R^{26}$ is fluoro, $R^4$ is trifluoromethyl and $R^2$ is chloro), may be prepared by reaction of a compound of formula (C IV), with a deprotonating agent followed by the addition of a source of the electrophile $Z^5$. A suitable deprotonating agent is lithium diisopropylamide and the deprotonation reaction suitably takes place under a nitrogen atmosphere and at a temperature of from -78°C to -23°C. A suitable solvent is tetrahydrofuran or diethyl ether. The source of the electrophile, $Z^5$ may then be added and the reaction allowed to warm to room temperature.

A suitable source of the electrophile $Z^5$ when it is chloro is N-chlorosuccinimide. A suitable source of the electrophile $Z^5$ when it is bromo is N-bromosuccinimide. A suitable source of the electrophile $Z^5$ when it is nitro is a nitrating agent such as nitronium tetrafluoroborate. A suitable source of the electrophile $Z^5$ when it is fluoro is the compound of formula (C V), In an alternative preparation of the compound of formula (C II), a compound of formula (C VI), may be reacted with a deprotonating agent such as lithium diisopropylamide as described above (except that the presence of the amide group requires two equivalents of the deprotonating agent) followed by the addition of a source of the electrophile $Z^5$ as described above,

followed finally by water to give a product of formula (C VII).

The compound of formula (C VII) can be converted to the compound of formula (C II) under the action of a dehydrating agent such as triphenylphosphorous in the presence of oxalyl chloride.

The compound of formula (C VI) may be prepared from the compound of formula (C IV) by treatment with concentrated sulphuric acid containing a small proportion of water.

The group $R^8$ may be introduced into a compound of formula (I) by replacement of a precursor group, $Z^8$ in a compound of formula (D), wherein Ar is a group of formula (D I).

$Z^8$ is preferably a halogen leaving group, for example bromine or is H which may subsequently be converted to halogen.

If the group Ar in the compound of formula (D) itself contains a halogen substituent such a bromine, competing reactions may lead to the formation of a mixture of products. Thus if it is desired to prepare a compound of formula (I) wherein any of $R^2$ to $R^5$ is bromine by this method, a precursor for the group $R^2$ to $R^5$ is preferably used. For example, if $R^2$ in the final product is to be bromine, the group $R^8$ may be introduced by replacement of a halogen leaving group $Z^8$ in a compound wherein $R^2$ is a nitro precursor group. The nitro precursor group $R^2$ may then be subsequently converted into a bromo group $R^2$ by reduction to an amino group followed by diazotisation and reaction with cuprous bromide as described previously.

Reaction of a compound of formula (D) wherein $Z^8$ is bromo with a compound of formula $H\text{-}C{\equiv}C\text{-}Y^1$ in the presence of a catalyst such as palladium metal or a palladium (II) salt and in the presence of a base such as triethylamine gives the compound of formula (I) wherein $R^8$ is the group $-C{\equiv}C\text{-}Y^1$.

Reaction of a compound of formula (D) wherein $Z^8$ is bromo with a compound of formula (D II), where $X^1$ is a leaving group such as trialkyl tin (for example tri-n-butyl tin) in the presence of a catalyst such as palladium metal or a palladium (II) salt gives a compound of formula (D III).

Hydrolysis of a compound of formula (D III) wherein $R^{16}$ is an alkoxy group, for example in the presence of aqueous hydrochloric acid, gives a compound of formula (D IV). Thus by choice of desired groups $R^{17}$ and $R^{18}$, a compound of formula (I) may be prepared wherein $R^8$ is $-CO\text{-}R^{25}$ and $R^{25}$ corresponds to $-CHR^{17}R^{18}$.

Oxidation of Compound (D III), for example using m-chloro-perbenzoic acid, yields a compound of formula (D V). Thus choice of groups $R^{16}$, $R^{17}$ and $R^{18}$ to correspond to the desired groups $R^{22}$, $R^{23}$ and $R^{24}$ respectively, will give a compound of formula (I) wherein $R^8$ is of formula (D VI) and A is oxygen.

Reaction of the compound of formula (D III) with a cyclopropylation agent will similarly give a product wherein $R^8$ is of formula (D VII) and A is $CH_2$.

The compound of formula (D) may be prepared by coupling of a compound of formula (II) with a compound of formula (III'), where $Z^8$ is a precursor group as defined previously, for example bromo or is hydrogen which may subsequently be converted into such a precursor group. The coupling takes place under the conditions described for the reaction of compound (II) with Compound (III).

Compounds of formula (III') can be prepared by general methods known in the art such as (a) condensation of a beta-ketoester of formula (VI), with thiourea or an S-alkyl isothiourea to give a compound of formula (VII), and subsequent desulphurisation with Raney nickel to give a compound of formula (III') where $R^6$ is hydrogen; or (b) condensation of the beta-ketoester with formamidine to give the compound of formula (III') where $R^6$ is hydrogen; or (c) halogenation of 4-($R^7$)-pyrimidin-6-one by conventional methods to give 5-halo-4-($R^7$)-pyrimidin-6-one (i.e. the compound of formula (III') wherein $Z^8$ is halogen).

More general conversion of a group $R^1$ to $R^9$ to a different such group may be carried out by conventional methods. In particular compounds of formula (I) wherein $R^2$, $R^4$, or any other substituent is nitro can be converted into the corresponding compound of formula (I) wherein that group is halo by reduction of the nitro group to an amino group to form, for example, a compound of formula (IV), wherein $R^3$ and $R^5$ are as defined in relation to formula (I) and Pm is as defined in formula (A) and $R^{2'}$ and $R^{4'}$ are amino or are equivalent to $R^2$ or $R^4$ as defined in relation to formula (I) respectively provided that at least one of $R^{2'}$ or $R^{4'}$ is amino; and thereafter converting the amino group $R^{2'}$, and/or $R^{4'}$ to halo.

Reduction of the nitro group to form a compound of formula (IV) may be carried out as previously described for a compound of formula (IIa).

Subsequent conversion of the amine to halogen may be carried out by reaction with t-butylnitrite and a copper halide salt such as copper (II) iodide. This step is suitably carried out in an organic solvent such as acetonitrile at low temperatures of from -20°C to +20°C preferably at about 0°C.

Compounds of formula (I) containing a $-SR^{13}$ group can be converted to the corresponding compounds of formula (I), where containing a $-SOR^{13}$ or $-SO_2R^{13}$ group by reaction with an oxidising agent such as m-chloro-perbenzoic acid.

Compounds of formula (I) where $R^4$ is trifluoromethylthio can be prepared from the corresponding

halogen derivatives, preferably where $R^4$ is iodine, by reaction with trifluoromethylthio copper.

Compound of formula (I) wherein $R^9$ is oxygen can be converted to sulphur by reaction with a thionating agent, such as Lawesson's reagent or $P_2S_5$, suitably at reflux.

Compounds of formula (II) wherein $R^1$ is cyano can be prepared by reacting a compound of formula (V), wherein $R^2$, $R^3$, $R^4$, $R^5$ and $R^{26}$ are as hereinbefore defined, and $R^{27}$ is halogen such as bromine, with a cyanide salt such as copper (I) cyanide in an organic solvent. The reaction is suitably carried out in an organic solvent such as quinoline, preferably at elevated temperature of 200 to 250 ° C. The reaction may optionally be carried out in the presence of a palladium catalyst.

Alternatively $R^{27}$ in formula (V) may be amino, and the reaction then requires the addition of t-butylnitrite in a suitable organic solvent such as acetonitrile. The reaction in this case is suitably effected at low temperatures of from 0 ° C to 10 ° C.

Compounds of formula (V) where $R^{27}$ is halogen are either known compounds or they can be prepared from known compounds by conventional methods.

Compounds of formula (V) where $R^{27}$ is amino are prepared by reducing the corresponding compound where $R^{27}$ is nitro.

The conversion of groups $R^1$-$R^9$ to different such groups may be carried out on the compound of formula (II) prior to coupling with the compound of formula (III) or (III'), if desired.

Compounds of formula (II) are either known compounds or they can be prepared from known compounds by conventional methods.

Suitable reactions for the preparation of compounds of formula (II) and for precursor compounds such as those of formula (D) above are illustrated in European Patent Application 0 398 499 (see for example Scheme 1 therein).

Other illustrative conversions of groups $R^2$ to $R^5$ are shown in schemes 6, 7 and 8 below.

Further details of the processes for preparation of the compounds may be ascertained from the Examples set out hereinafter.

The compounds of formula (I) may be used to combat and control infestations of insect pests and also other invertebrate pests, for example, acarine pests. The insect and acarine pests which may be combated and controlled by the use of the invention compounds include those pests associated with agriculture (which term includes the growing of crops for food and fibre products, horticulture and animal husbandry), forestry, the storage of products of vegetable origin, such as fruit, grain and timber, and also those pests associated with the transmission of diseases of man and animals.

In order to apply the compounds to the locus of the pests they are usually formulated into compositions which include in addition to the insecticidally active ingredient or ingredients of formula I suitable inert diluent or carrier materials, and/or surface active agents. The compositions may also comprise another pesticidal material, for example another insecticide or acaricide, or a fungicide, or may also comprise an insecticide synergist, such as for example dodecyl imidazole, safroxan, or piperonyl butoxide.

The compositions may be in the form of solid preparations that may be applied diluted or undiluted.

Solid compositions that may be applied undiluted may be in the form of dusting powders wherein the active ingredient is mixed with a solid diluent or carrier, eg kaolin, bentonite, kieselguhr, silica or talc. Or the solid composition may be in the form of granules wherein the active ingredient is absorbed on a non-porous granular material, for example, calcium carbonate, or may be impregnated in a porous granular material, for example, pumice or gypsum.

Solid compositions that may be applied diluted may be in the form of wettable powders wherein the active ingredient is mixed with a solid diluent or carrier, such as kaolin, kieselguhr or silica and appropriate surface acting agents or they may be in the form of water dispersible granules, wherein the active ingredient is mixed with a solid diluent or carrier, for example, kaolin, kieselguhr or silica and an appropriate surface acting agent, and then granulated.

Alternatively, the compositions may be in the form of liquid preparations to be used as dips, sprays or aerosol dispersions or non-aqueous solutions of the active ingredient and are usually diluted before application.

Aqueous dispersions of the active ingredient which may be applied diluted may be in the form of suspension concentrates wherein the active ingredient is dispersed in an aqueous media. These compositions contain dispersing/ wetting agents and one or more stabilising agents, for example, bentonite clays and/or polysaccharide gels. Additional further components may be included such as antifreeze agents, for example, ethylene glycol, propylene glycol or salts, and biocides, for example, Proxel GXL (1,2-benzisothiazolin-3-one).

Other aqueous dispersions of the active ingredient may be in the form of microcapsule suspensions wherein the active ingredient is encapsulated, as a high strength water immiscible solution, with a polymer

and the subsequent microcapsules are dispersed in aqueous media. The microencapsulation technique used may be of the type described in the patent literature. These compositions contain dispersing/wetting agents and one or more stabilising agents, for example, bentonite clays and/or polysaccharide gels. Additional further components may be included such as anti-freeze agents and biocides as previously described.

Other aqueous dispersions of the active ingredient may be in the form of oil in water emulsions wherein the active ingredient is dissolved in a suitable solvent, for example, an aromatic hydrocarbon such as trimethylbenzene or a ketonic solvent such as di-hydroisophorone alone with one or more emulsifying agents and then emulsifying the solution so obtained into water which may contain further surface active agents. Other suitable organic solvents are ethylene dichloride, toluene, kerosene, white oil, methylnaphthalene, xylenes, trichloroethylene, vegetable oils, N-methyl-2-pyrrolidone and isophorone.

Alternatively liquid compositions may be in the form of non-aqueous solutions to be used diluted or undiluted as sprays or aerosol fogs.

Non-aqueous preparations that may be applied undiluted may be in the form of low volume or ultra low volume concentrates wherein the active ingredient is dissolved in a suitable solvent or mixture of solvents, for example, an aromatic hydrocarbon such as trimethylbenzene or aliphatic hydrocarbon such as kerosene. Other suitable solvents are isophorone, di-hydroisophorone, toluene, xylenes, methylnapthalenes, N-methylpyrrolidone, mineral oil and vegetable oils. These preparations are optionally diluted before application with paraffinic solvents, such as diesel oil.

Other non-aqueous preparations may be in the form of emulsifiable concentrates wherein the active ingredient is dissolved in a suitable solvent, for example, trimethyl-benzenes or methylcyclohexanone, with one or more emulsifying agents. Other suitable solvents are as previously described. These preparations are diluted in water to form aqueous dispersions before application.

Further formulation types may include preparations for special use such as aerosols wherein the composition will contain the active ingredient or ingredients, a propellant and an inert diluent, for example, odourless kerosenes or alkylated benzenes. In a preferred form, aerosol compositions may contain from 0.005% to 4% of active ingredient or ingredients, the remainder of the composition may be aqueous based in which an aqueous component is dispersed in a solution of active ingredient in a solvent, such as previously described, and a propellant by using one or more surface active agents. Aerosol compositions may optionally incorporate other additives, for example, knockdown agents, synergists, perfumes and corrosion inhibitors.

Other formulations for special purposes may be in the form of ready for use sprays wherein the active ingredient is dissolved in a solvent, for example, odourless kerosenes and alkylated benzenes and applied through a hard pump device to be used as a residual spray. These compositions may optionally incorporate other additives such as knockdown agents, synergists and perfumes.

Wetting agents, dispersing agents and emulsifying agents may be of the cationic, anionic or non-ionic type. Suitable agents of the cationic type include, for example, quaternary ammonium compounds, for example cetyltrimethyl ammonium bromide. Suitable agents of the anionic type include, for example, soaps, salts of aliphatic monoesters of sulphuric acid, for example sodium lauryl sulphate, salts of sulphonated aromatic compounds, for example sodium dodecylbenzenesulphonate, sodium, calcium or ammonium lignosulphonate, or butylnaphthalene sulphonate, and a mixture of the sodium salts of diisopropyl- and triisopropylnaphthalene sulphonates. Suitable agents of the non-ionic type include, for example, the condensation products of ethylene oxide with fatty alcohols such as oleyl alcohol or cetyl alcohol, or with alkyl phenols such as octyl phenol, nonyl phenol and octyl cresol. Other non-ionic agents are the partial esters derived from long chain fatty acids and hexitol anhydrides, the condensation products of the said partial esters with ethylene oxide, and the lecithins.

The compositions which are to be used in the form of aqueous dispersions or emulsions are generally supplied in the form of a concentrate containing a high proportion of the active ingredient or ingredients, the said concentrate to be diluted with water before use. These concentrates are often required to withstand storage for prolonged periods and after such storage, to be capable of dilution with water to form aqueous preparations which remain homogeneous for a sufficient time to enable them to be applied by conventional spray equipment. The concentrates may contain 10-85% by weight of the active ingredient or ingredients. When diluted to form aqueous preparations such preparations may contain varying amounts of the active ingredient depending upon the purpose for which they are to be used. For agricultural or horticultural purposes, an aqueous preparation containing between 0.0001% and 0.1% by weight of the active ingredient (approximately equivalent to from 5-2000g/ha) is particularly useful.

In use the compositions are applied to the pests, to the locus of the pests, to the habitat of the pests, or to growing plants liable to infestation by the pests, by any of the known means of applying pesticidal

compositions, for example, by dusting or spraying.

The compounds of the invention may be the sole active ingredient of the composition or they may be admixed with one or more additional active ingredients such as insecticides, insecticide synergists, herbicides, fungicides or plant growth regulators where appropriate.

Suitable additional active ingredients for inclusion in admixture with the compounds of the invention may be compounds which will broaden the spectrum of activity of the compounds of the invention or increase their persistence in the location of the pest. They may synergise the activity of the compound of the invention or complement the activity for example by increasing the speed of effect, improving knockdown or overcoming repellency. Additionally multi-component mixtures of this type may help to overcome or prevent the development of resistance to individual components.

The particular insecticide, herbicide or fungicide included in the mixture will depend upon its intended utility and the type of complementary action required. Examples of suitable insecticides include the following:

a) Pyrethroids such as permethrin, esfenvalerate, deltamethrin, cyhalothrin in particular lambda- cyhalothrin, biphenthrin, fenpropathrin, cyfluthrin, tefluthrin, fish safe pyrethroids for example ethofenprox, natural pyrethrin, tetramethrin, s-bioallethrin, fenfluthrin, prallethrin and 5-benzyl-3-furylmethyl-(E)-(1R,3S)-2,2-dimethyl-3-(2-oxothiolan-3-ylidenemethyl)-cyclopropane carboxylate;

b) Organophosphates such as profenofos, sulprofos, methyl parathion, azinphos-methyl, demeton-s-methyl, heptenophos, thiometon, fenamiphos, monocrotophos, profenophos, triazophos, methamidophos, dimethoate, phosphamidon, malathion, chloropyrifos, phosalone, fensulfothion, fonofos, phorate, phoxim, pyrimiphos-methyl, fenitrothion or diazionon;

c) Carbamates (including aryl carbamates) such as pirimicarb, cloethocarb, carbofuran, ethiofencarb, aldicarb, thiofurox, carbosulfan, beniocarb, fenobucarb, propoxur or oxamyl;

d) Benzoyl ureas such as triflumeron, or chlorofluazuron;

e) Organic tin compounds such as cyhexatin, fenbutatin oxide, azocyclotin;

f) Macrolides such as avermectins or milbemyins, for example such as abamectin, avermectin, and milbemycin;

g) Hormones such as pheromones;

h) Organochlorine compounds such as benzene hexachloride, DDT, chlordane or dieldrin.

i) Amidines, such as chlordimeform or amitraz.

In addition to the major chemical classes of insecticide listed above, other insecticides having particular targets may be employed in the mixture if appropriate for the intended utility of the mixture. For instance selective insecticides for particular crops, for example stemborer specific insecticides for use in rice such as cartap or buprofezin can be employed. Alternatively insecticides specific for particular insect species/stages for example ovo-larvicides such as clofentezine, flubenzimine, hexythiazox and tetradifon, moltilicides such as dicofol or propargite, acaricides such as bromopropylate, chlorobenzilate, or growth regulators such as hydramethylon, cyromazin, methoprene, chlorofluazuron and diflubenzuron may also be included in the compositions.

Examples of suitable insecticide synergists for use in the compositions include piperonyl butoxide, sesamex, and dodecyl imidazole.

Suitable herbicides, fungicides and plant-growth regulators for inclusion in the compositions will depend upon the intended target and the effect required.

An example of a rice selective herbicides which can be included is propanil, an example of a plant growth regulator for use in cotton is "Pix", and examples of fungicides for use in rice include blasticides such as blasticidin-S.

The ratio of the compound of the invention to the other active ingredient in the composition will depend upon a number of factors including type of target, effect required from the mixture etc.

However in general, the additional active ingredient of the composition will be applied at about the rate as it is usually employed, or at a slightly lower rate if synergism occurs.

The compounds of formula I and compositions comprising them have shown themselves active against a variety of insect and other invertebrate pests. They are particularly useful in controlling public health pests such as flies and cockroaches. They may also be active against organophosphate and pyrethroid resistant strains of pests such as houseflies (Musca domestica). They may be effective in combating both susceptible and resistant strains of the pests in their adult, larval and intermediate stages of growth, and may be applied to the infested host animal by topical, oral or parenteral administration.

The following Preparations and Examples illustrate various aspects of the invention. In the Preparations and Examples the compounds were identified and characterised by means of the melting points, nuclear magnetic resonance spectroscopy (in $CDCl_3$ or $d_6$DMSO, using a Jeol GSX machine at 270 mHz), mass

spectroscopy (using a VG TRIO 1 machine) or infra red spectroscopy (using a Perkin-Elmer Model 881).

Precursor compounds of formula (D) wherein $R^1$ is cyano and $R^6$ is hydrogen are listed in Table A1 below. The preparation of these compounsds is disclosed in European Patant Application 0 398 499. Those skilled in the art will readily appreciate how compounds listed in Tables A I may be converted into compounds of the present invention using the conversion techniques described and illustrated above. In particular, where $Z^8$ is not halogen in Table A 1, $Z^8$ may be converted to halogen and thereafter into a group $R^8$ as defined above. Groups $R^2$ which are halogen or nitro may be converted into other groups $R^2$ as described and groups $R^5$ which are halogen may be converted into other groups $R^5$ as described. Alternatively, compounds wherein $Z^8$ in formula (D) is halo may be prepared in a corresponding manner to those listed in Table A 1 wherein $Z^8$ is hydrogen, except that the halogen ($R^8$) may be introduced prior to the coupling reaction of a compound of formula (II) and a compound of formula (III).

TABLE A I

| COMPOUND NO. | $R^7$ | $Z^8$ | $R^2$ | $R^3$ | $R^4$ | $R^5$ | $R^9$ |
|---|---|---|---|---|---|---|---|
| A1 | $CF_3$ | H | H | H | $CF_3$ | H | 0 |
| A2 | $CF_3$ | H | Cl | H | $CF_3$ | H | 0 |
| A3 | $C_2F_5$ | H | H | H | $CF_3$ | H | 0 |
| A4 | $C_2F_5$ | H | Cl | H | $CF_3$ | H | 0 |
| A5 | $CF_3$ | H | Br | H | $CF_3$ | H | 0 |
| A6 | $C_2F_5$ | H | Br | H | $CF_3$ | H | 0 |
| A7 | $CF_3$ | H | $NO_2$ | H | $CF_3$ | H | 0 |
| A8 | $CF_3$ | Br | Cl | H | $CF_3$ | H | 0 |
| A9 | $^nC_3F_7$ | H | Cl | H | $CF_3$ | H | 0 |

TABLE A I (continued)

| COMPOUND NO. | $R^7$ | $Z^8$ | $R^2$ | $R^3$ | $R^4$ | $R^5$ | $R^9$ |
|---|---|---|---|---|---|---|---|
| A10 | $CF_3$ | H | CN | H | $CF_3$ | H | O |
| A11 | $C_2F_5$ | H | CN | H | $CF_3$ | H | O |
| A12 | $C_2F_5$ | Br | Br | H | $CF_3$ | H | O |
| A13 | $CF_3$ | H | F | H | $CF_3$ | H | O |
| A19 | $CF_3$ | H | Cl | H | $CF_3$ | H | S |
| A21 | $C_2F_5$ | H | F | H | $CF_3$ | H | O |
| A22 | $C_2F_5$ | H | Cl | H | $OCF_3$ | H | O |

Preparation 1

The preparation of 5-acetyl-4-trifluoromethylpyrimidin-6-one is illustrated. Sodium hydride (0.99gms of 55% oil dispersion) was washed with hexane and suspended in dry tetrahydrofuran (100mls). With stirring at room temperature under a nitrogen atmosphere 5-bromo-4-trifluoromethyl-pyrimidin-6-one (5.0gms) was added over 10 minutes. The resulting solution was stirred at room temperature for 30 minutes and cooled to -78°C. A solution of tert-butyl lithium (26.6mls of 1.7M) in pentane was added dropwise over 30 minutes keeping the temperature below -70°C. The bright yellow solution was stirred for 1 hour at -78°C and then acetic anhydride (19.4mls) was added rapidly. The mixture was allowed to warm to room temperature over 3 hours. The mixture was poured into 2M hydrochloric acid and saturated with ethyl acetate. The combined extracts were washed with brine, dried over magnesium sulphate and concentrated to a sticky yellow solid. Trituration with ether-hexane and washing with hexane gave 5-acetyl-4-trifluoromethyl-pyrimidin-6-one. Melting point: 176.2-180.8°C (decomposition) $^1H$ NMR $\delta(d_6 DMSO)$: 8.50(s,1H), 2.48(s,3H).

Preparation 2

The preparation of 5-(2-hydroxyisopropyl)-4-trifluoromethyl-pyrimidin-6-one is illustrated.

Methyl magnesium bromide (2.7mls of 3M solution in ether) was added dropwise to a solution of 5-acetyl-4-trifluoromethylpyrimidin-6-one (Preparation 1, 0.75gms) in dry tetrahydrofuran (20mls) with stirring under nitrogen keeping the temperature below 5°C. The resulting green mixture was stirred at 0°C for 1½ hours, allowed to warm to room temperature and stirred for 1 hour and then heated at 50°C for ½ hour. After cooling and pouring into 2M hydrochloric acid the mixture was extracted with ethyl acetate. the combined extracts were washed with brine, dried over magnesium sulphate and concentrated to a yellow gum. The product was isolated by flash chromatography on silica eluting with ethyl acetate (50-80%) in hexane followed by HPLC on silica eluting with 80% ethyl acetate in hexane to give 5-(2-hydroxyisopropyl)-4-trifluoromethyl pyrimidin-6-one. Melting point: 139.4-142.2°C; $^1H$ NMR $\delta(CDCl_3)$: 8.21(s,1H), 5.03(brs,1H).

EXAMPLE 1

This Example illustrates the preparation of 1-(2-chloro-6-cyano-4-trifluoromethylphenyl)-4-trifluoromethyl-5-vinylpyrimidin-6-one (compound No. 1 in Table I).

A solution of 1-(2-chloro-6-cyano-4-trifluoromethylphenyl)-5-bromo-4-trifluoromethylpyrimidin-6-one (Compound No A8 in Table A1), (2g), vinyl tri-n-butyltin (1.44ml) and bis-triphenylphosphine-palladium (II) chloride (63mg) in dry dimethylformamide (20ml), was heated to 70°C, under an atmosphere of nitrogen for

7.5 hours. After cooling to ambient temperature, the reaction mixture was poured into saturated aqueous potassium fluoride solution (150ml). Ethyl acetate (100ml) was added, and after vigorous mixing for 10 minutes, the mixture was filtered through celite, and the organic layer separated. The aqueous layer was re-extracted with ethyl acetate, and the combined organic layers dried over magnesium sulphate. Filtration, followed by evaporation of the solvent, under reduced pressure, gave a yellow oil, which was subjected to column chromatography on silica gel, using hexane containing ethyl acetate (10-15% by volume) as eluent. The desired fractions were concentrated, by evaporation of the solvent, under reduced pressure, and the resultant yellow solid washed with hexane to give 1-(2-chloro-6-cyano-4-trifluoromethylphenyl)-4-trifluoromethyl-5-vinylpyrimidin-6-one. Melting Point: 149-151 °C; $^1$H NMR $\delta(CDCl_3)$ 8.15(1H,s); 8.07(1H,s); 7.95(1H,s); 6.85-6.60(2H,m); 5.85(1H,dd).

EXAMPLE 2

This Example illustrates the preparation of 1-(2-chloro-6-cyano-4-trifluoromethylphenyl)-5-(1-ethoxyvinyl)-4-trifluoromethyl-pyrimidin-6-one (compound No. 2 in Table I).

1-(2-Chloro-6-cyano-4-trifluoromethylphenyl)-5-bromo-4-trifluoromethylpyrimidin-6-one was reacted with (1-ethoxyvinyl)tri-n-butyltin, in the presence of bis-(triphenylphosphine)-palladium (II) chloride in a manner analogous to that described in Example 1, to give 1-(2-chloro-6-cyano-4-trifluoromethylphenyl)-5-(1-ethoxyvinyl)-4-trifluoromethyl-pyrimidin-6-one. Melting Point: 100-102 °C; $^1$H NMR $\delta(CDCl_3)$ 8.14(1H,s); 8.03-(1H,s); and 8.03(1H,s); 4.58(1H,d); 4.40(1H,d); 3.90(2H,q); 1.38(3H,t).

EXAMPLE 3

This Example illustrated the preparation of 1-(2-chloro-6-cyano-4-trifluorormethylphenyl)-5-acetyl-4-trifluromethylpyrimidin-6-one (compound No. 3 in Table I).

1-(2-chloro-6-cyano-4-trifluoromethylphenyl)-5-(1-ethoxylvinyl)-4-trifluoromethyylpyrimidin-6-one (the product of Example 2) (2.5g) in a mixture of acetone (20ml) and 1 molar aqueous hydrochloric acid (8ml), was heated to 50 °C for a period of 3 hours. The acetone was then removed by evaporation, under reduced pressure, and the residue was dissolved in water and extracted with ethyl acetate. The combined organic extracts were washed with water and brine, dried over anhydrous magnesium sulphate, and concentrated by evaporation of the solvent, under reduced pressure, to give a white solid. Trituration with hexane gave 1-(2-chloro-6-cyano-4-trifluoromethylphenyl)-5-acetyl-4-trifluoromethylpyrimdin-6-one: Melting Point: 154.2-156.2 °C. $^1$H NMR $\delta(CDCl_3)$ 8.16(1H,s); 8.12(1H,s); 8.08(1H,s); 2.55(3H,s).

EXAMPLE 4

This Example illustrates the preparation of 1-(2-chloro-6-cyano-4-trifluoromethylphenyl)-5-(1-methoxyvinyl)-4-trifluoromethyl-pyrimidin-6-one (Compound No.4 in Table 1).

1-(2-chloro-6-cyano-4-trifluoromethylphenyl)-5-bromo-4-trifluoromethylpyrimidin-6-one was reacted with (1-methoxyvinyl) tri-n-butyltin (prepared by the method of Organometallics, 1(6), 830-833, 1982) in the presence of bis-(triphenylphosphine)-palladium (II) chloride in a manner analogous to that described in Example 1 to give 1-(2-chloro-6-cyano-4-trifluoromethylphenyl)-5-(1-methoxyvinyl)-4-trifluoromethylpyrimidin-6-one. Melting Point: 115.8 - 117.8 °C, $^1$H NMR $\delta$ $(CDCl_3)$: 8.13(d,1H); 8.05(s,2H); 4.44(d,1H); 4.63(d,1H); 3.72(s,3H).

EXAMPLE 5

This Example illustrates the preparation of 1-(2-chloro-6-cyano-4-trifluoromethylphenyl)-5-(2-propenyl)-4-trifluoromethylpyrimidin-6-one (Compound No 5 in Table 1).

Stage 1

1-(2-chloro-6-cyano-4-trifluoromethylphenyl)-5-(2-hydroxyisopropyl)-4-trifluoromethylpyrimidin-6-one was prepared from 3-chloro-4-fluoro-5-cyano-trifluoromethylbenzene and 5-(2-hydroxyisopropyl)-4-trifluoromethyl-pyrimidin-6-one (Preparation 2) according to the general method disclosed in EP 0 398 499 for the preparation of Compound A1 in Table A1. Melting Point: 125.6 - 127.6 °C, $^1$H NMR $\delta(CDCl_3)$: 8.14-(s,1H); 8.05(s,1H); 7.99(s,1H); 3.45(s,1H); 1.75(d,6H).

Stage 2

1-(2-chloro-6-cyano-4-trifluoromethylphenyl)-5-(2-hydroxy isopropyl)-4-trifluoromethylpyrimidin-6-one (174mgms) (from Stage 1) and paratoluene sulphonic acid monohydrate (20mgms) were refluxed in toluene (10mls) under nitrogen in a Dean-Stark apparatus for 6 hours. The solution was cooled, diluted with ethyl acetate and washed with water and brine, dried over magnesium sulphate and concentrated to an off-white solid which was purified by HPLC eluting with 20% ethyl acetate in hexane to give (1-(2-chloro-6-cyano-4-trifluoromethylphenyl)-5-(2-propenyl)-4-trifluoromethyl pyrimidin-6-one. Melting Point: 135.6-137.6° C, $^1$H NMR $\delta$($CDCl_3$); 8.14(s,1H); 8.05(s,1H); 8.01(s,1H); 5.40(s,1H); 5.10(s,1H); 2.07(s,3H).

EXAMPLE 6

This Example illustrates the preparation of 1-(2-carboxamido-6-chloro-4-trifluoromethylphenyl)-5-(2-propenyl)-4-trifluoromethyl-pyrimidin-6-one (compound No. 6 in Table 1).

1-(2-chloro-6-cyano-4-trifluoromethylphenyl)-5-(2-hydroxyisopropyl)-trifluoromethylpyrimidin-6-one (50mgms, Stage 1 of Example 5) was stirred at room temperature in concentrated sulphuric acid (2mls). The solution was poured into water and the mixture extracted with ethyl acetate. The combined extracts were washed with brine, dried with magnesium sulphate and concentrated to a white solid containing two components. These were separated by flash chromatography on silica eluting with ethyl acetate (20-60%) in hexane to give 1-(2-chloro-6-cyano-4-trifluoromethylphenyl)-5-(2-chloro-6-cyano-4-trifluoromethylphenyl)-5-(2-propenyl)-4-trifluormethylpyrmidin-6-one (minor component, the product of Example 5) and 1-(2-carboxamido-6-chloro-4-trifluoromethylphenyl)-5-(2-propenyl)-4-trifluoromethylpyrimidin-6-one. Melting point: 199.6-201.6° C. $^1$H NMR $\delta$ ($CDCl_3$): 7.96(s,1H), 7.82(s,1H), 5.78(brs,1H), 6.44(brs,1H), 5.03(s,1H), 5.35(s,1H), 2,01(s,1H), 8.01(s,1H).

EXAMPLE 7

The activity of the compounds of formula (I) was determined using a variety of pests. The pests were treated with a liquid composition containing 500 parts per million (ppm) by weight of the compound unless otherwise stated. The compositions were made by dissolving the compound in acetone and diluting the solutions with water containing 0.01% by weight of a wetting agent sold under the trade name "SYNPERONIC" NX until the liquid composition contained the required concentration of the compound. "SYNPERONIC" is a Registered Trade Mark.

The test procedure adopted with regard to each pest was basically the same and comprised supporting a number of the pests on a medium which was usually a host plant or a foodstuff on which the pests feed, and treating either or both the medium and the pests with the compositions. The mortality of the pests was then assessed at periods usually varying from one to three days after the treatment.

The results of the tests are presented in Table II for each of the compounds at the rate in parts per million given in the second column. The results indicate a grading of mortality designated as A, B or C wherein A indicates 80-100% mortality, B indicates 50-79% mortality and C indicates less than 50% mortality.

Information regarding the pest species, the support medium or food, and the type and duration of the test is given in Table III. The pest species is designated by a letter code.

TABLE II

| COMPOUND | RATE OF APPLICATION ppm | SPECIES MD (see Table III) | SPECIES BG (see Table III) |
|---|---|---|---|
| 1 | 500 | A | A |
| 2 | 500 | A | A |
| 3 | 500 | A | A |
| 4 | 500 | A | A |
| 5 | 500 | A | A |

TABLE III

| CODE LETTERS | TEST SPECIES | SUPPORT MEDIUM/FOOD | TYPE OF TEST | DURATION (days) |
|---|---|---|---|---|
| MD | Musca domestica (houseflies - adults) | Cotton wool/sugar | Contact | 2 |
| BC | Blattella germanica (cockroach nymphs) | Plastic pot/calf weaner pellets | Contact | 3 |

CHEMICAL FORMULAE
(in description)

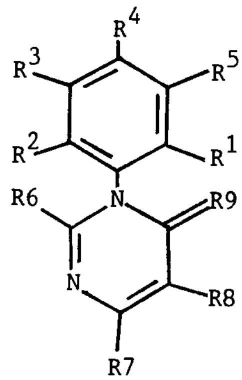

(I)

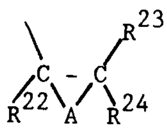

(Ia)

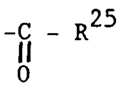

(Ib)

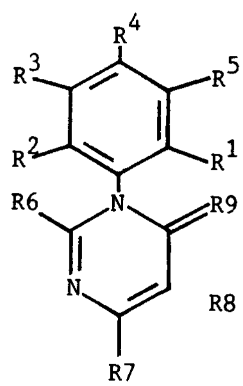

(I')

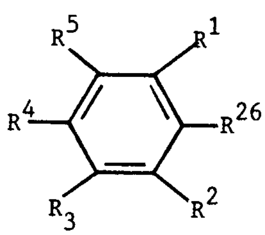

(II)

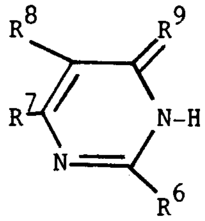

(III)

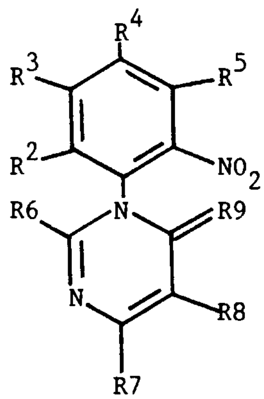

(Ic)

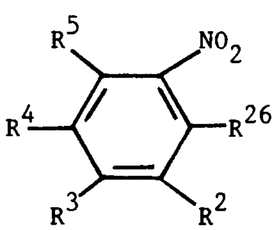

(IIa)

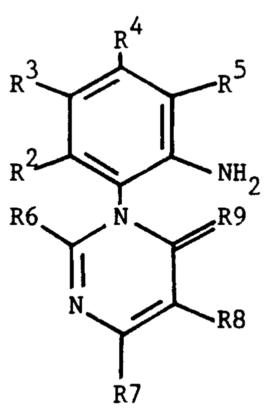

(Id)

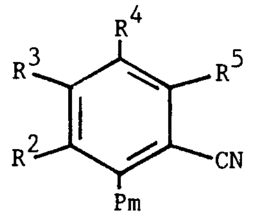

(A)

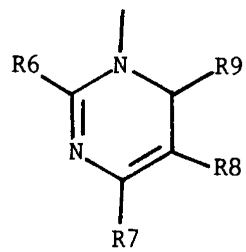

(A')

Scheme 1

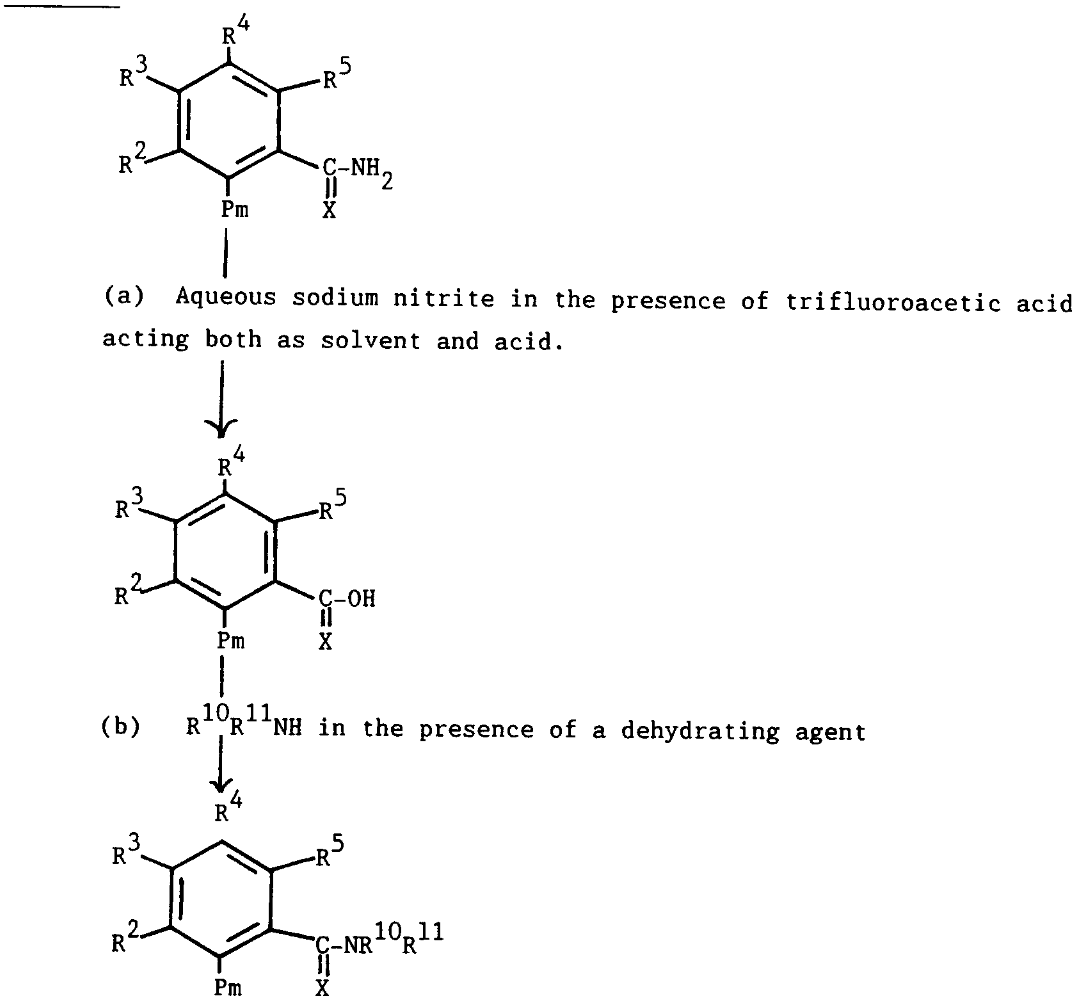

(a) Aqueous sodium nitrite in the presence of trifluoroacetic acid acting both as solvent and acid.
(b) $R^{10}R^{11}NH$ in the presence of a dehydrating agent

Scheme 2

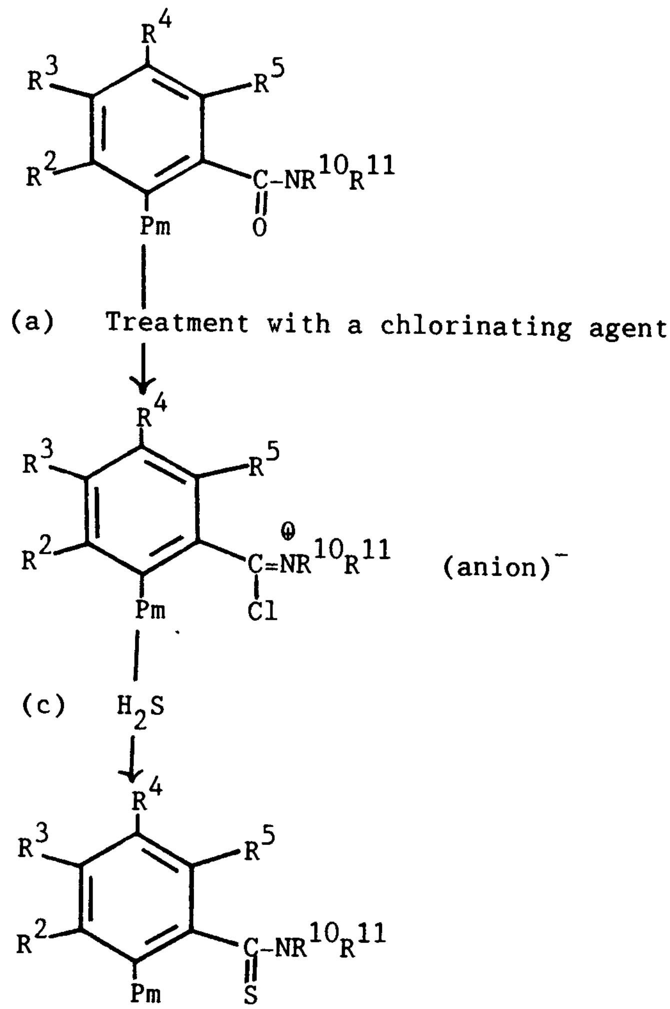

Scheme 2′

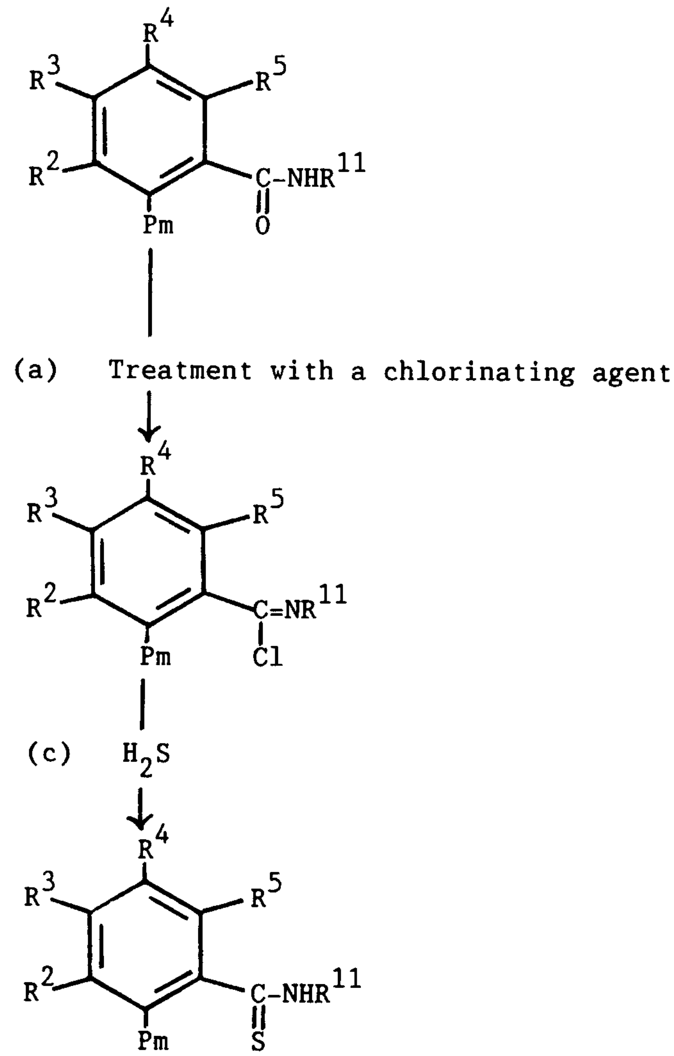


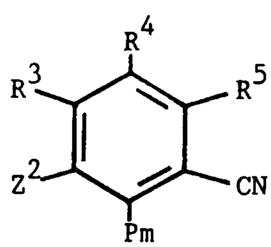

(B)

<u>*Scheme 3*</u>

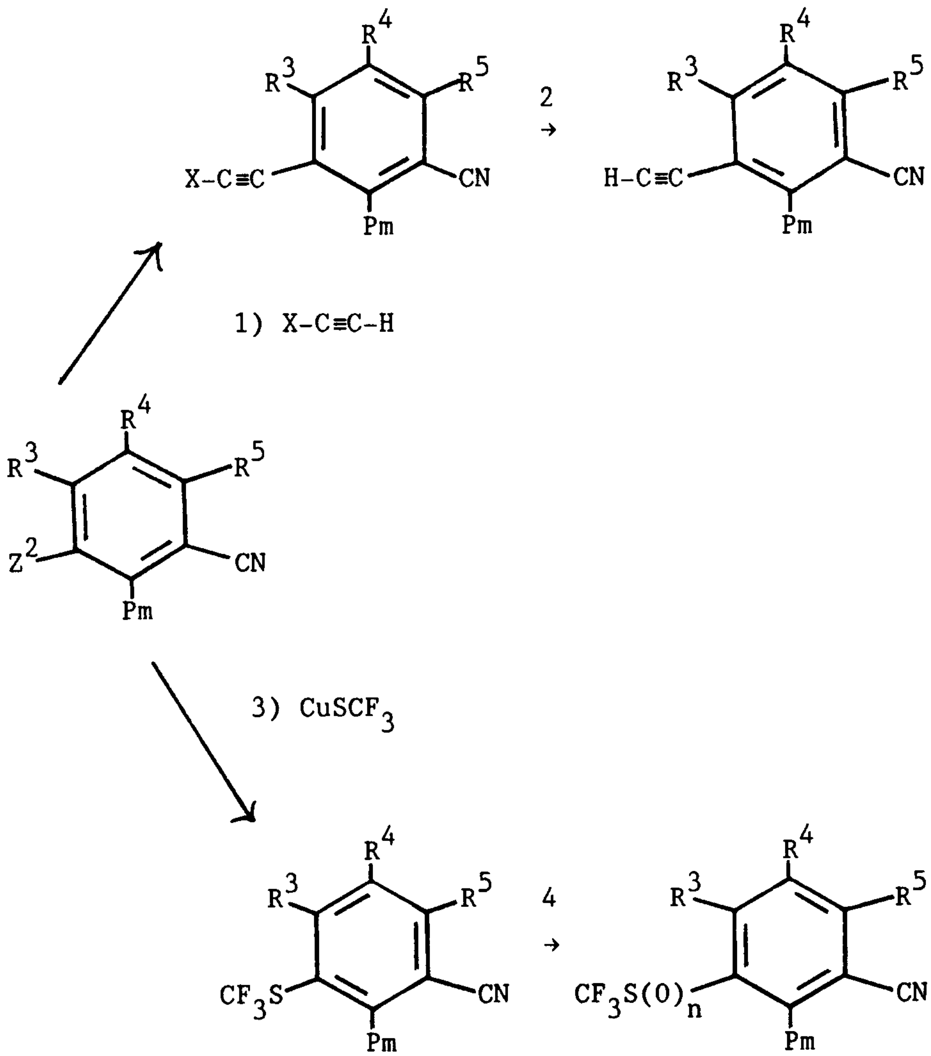

Scheme 4

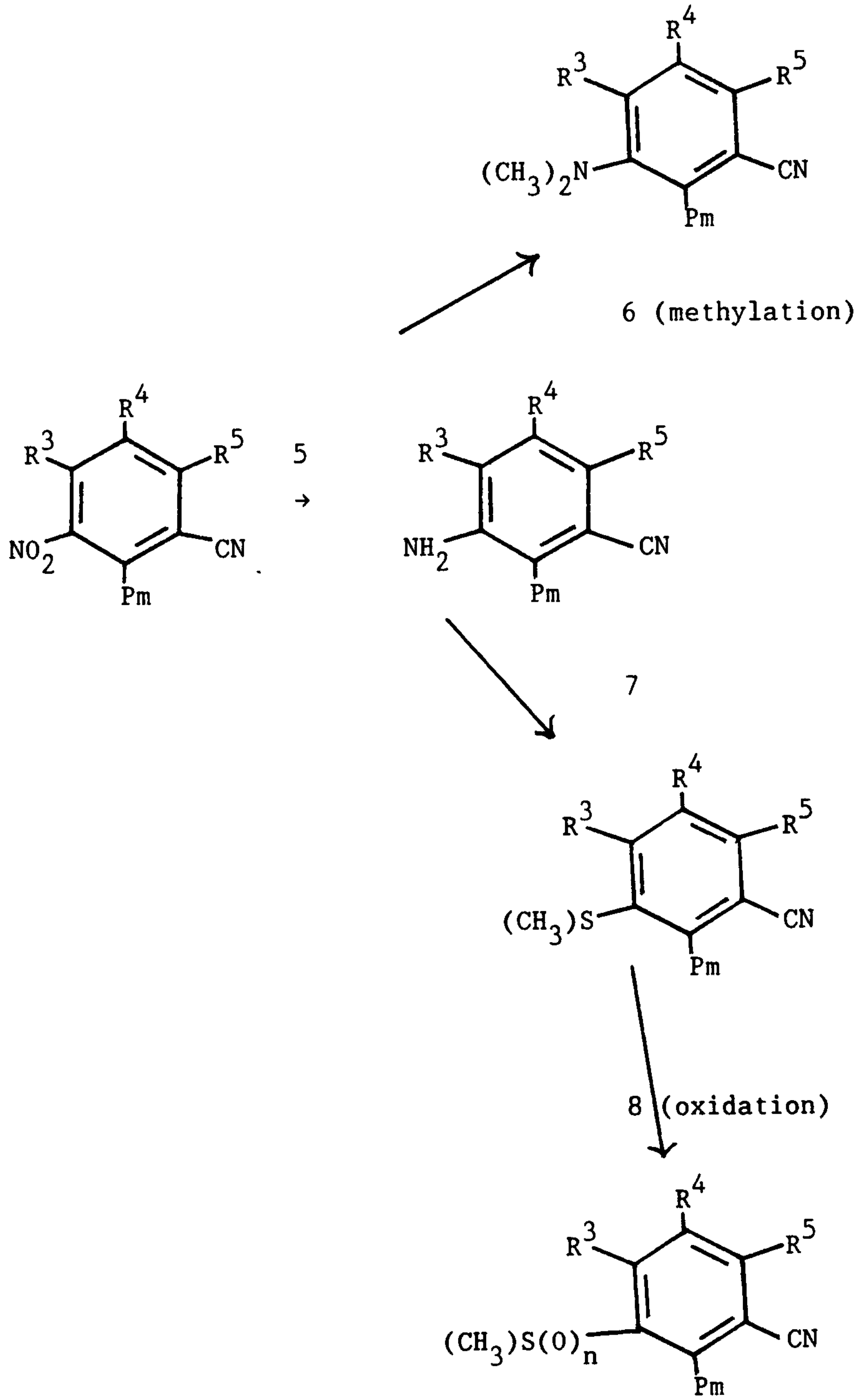

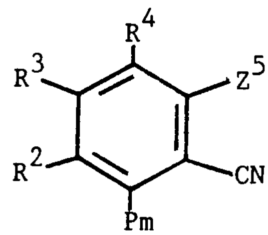

(C)

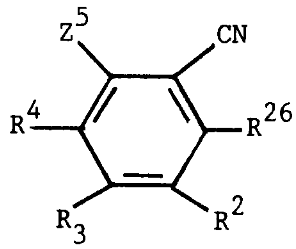

(C II)

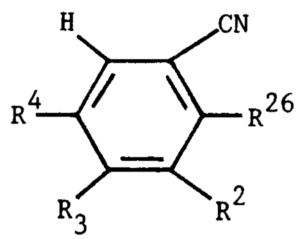

(C IV)

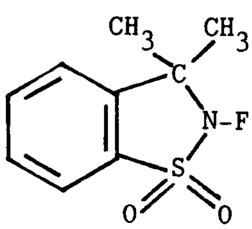

(C V)

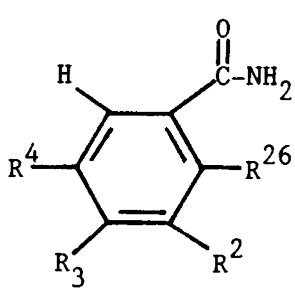

(C VI)

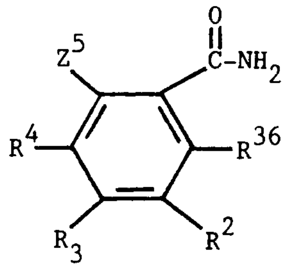

(C VII)

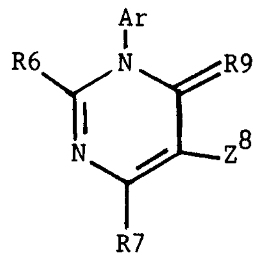

(D)

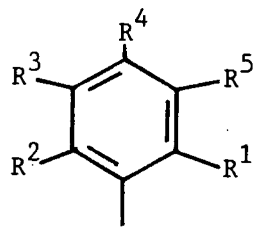

(D I)

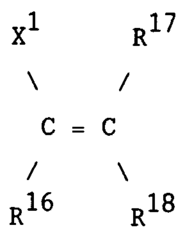

(D II)

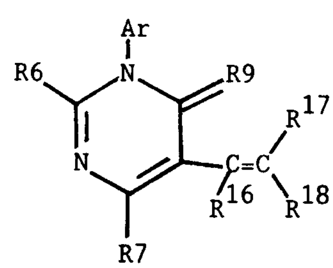

(D III)

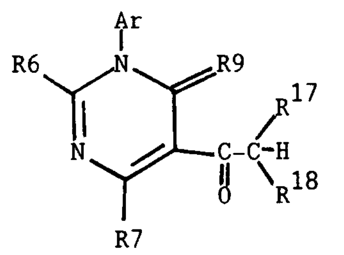

(D IV)

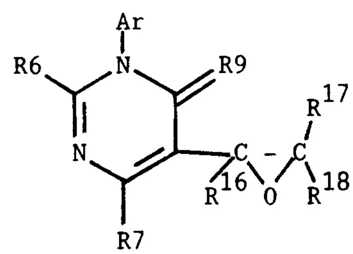

(D V)

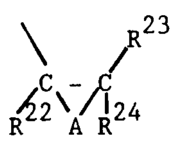

(D VI)

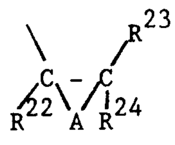

(D VII)

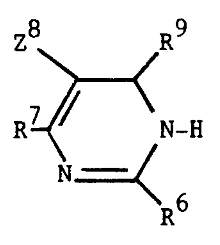

(III')

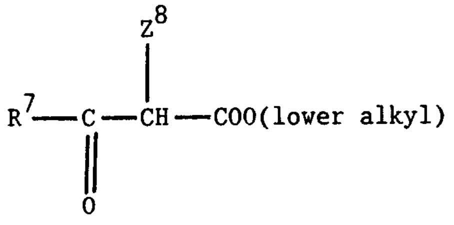

(VI)

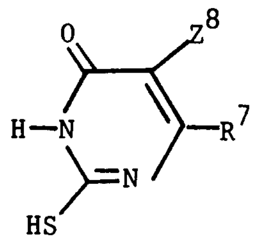

(VII)

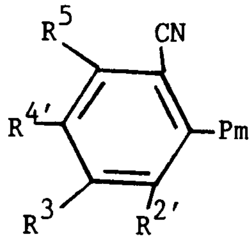

(IV)

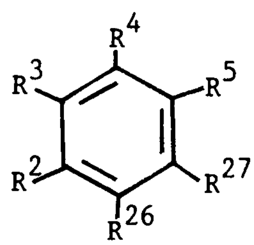

(V)

Scheme 6

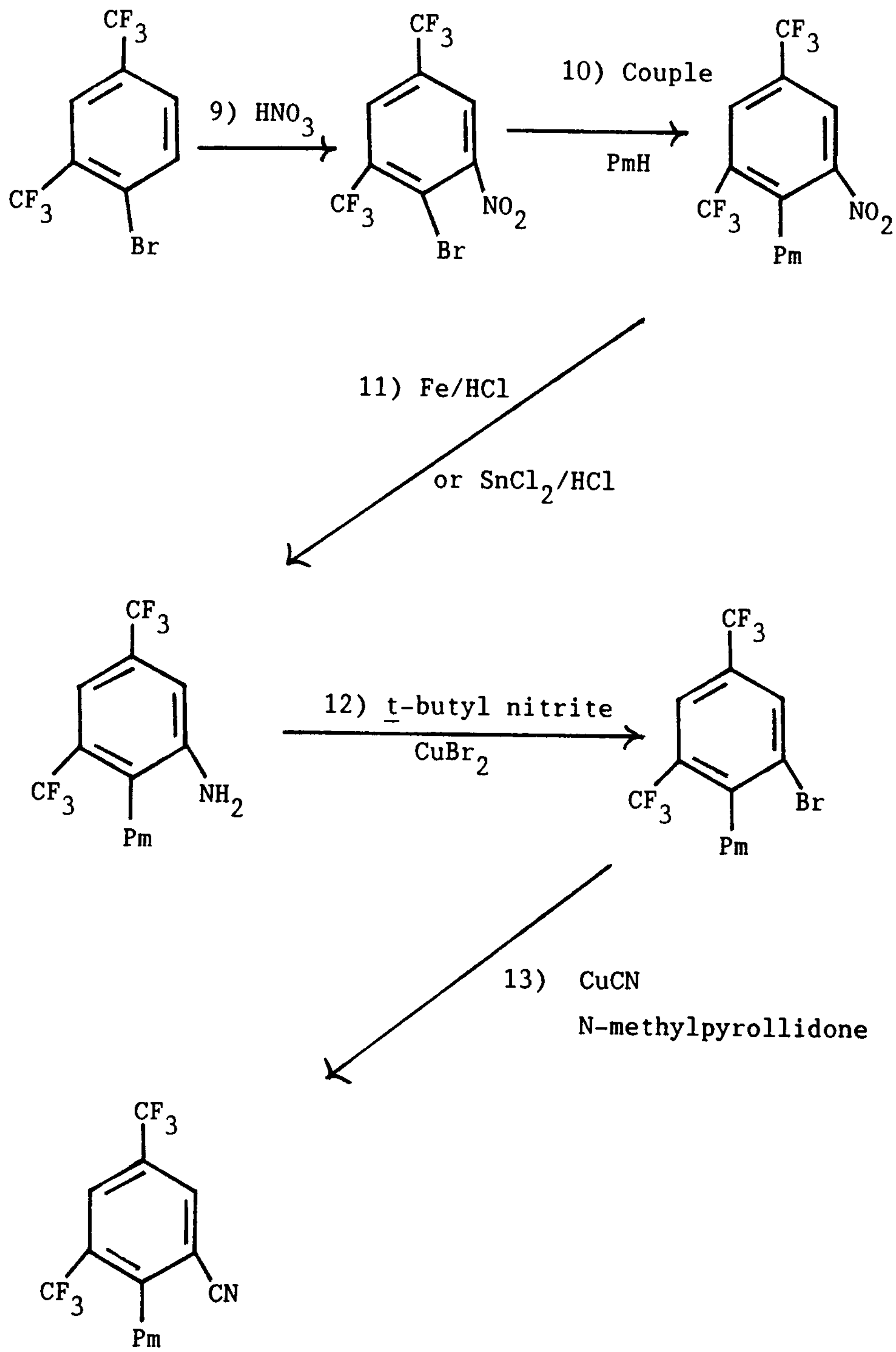
9) HNO3
10) Couple
PmH
11) Fe/HCl
or SnCl2/HCl
12) t-butyl nitrite
CuBr2
13) CuCN
N-methylpyrollidone

Scheme 7

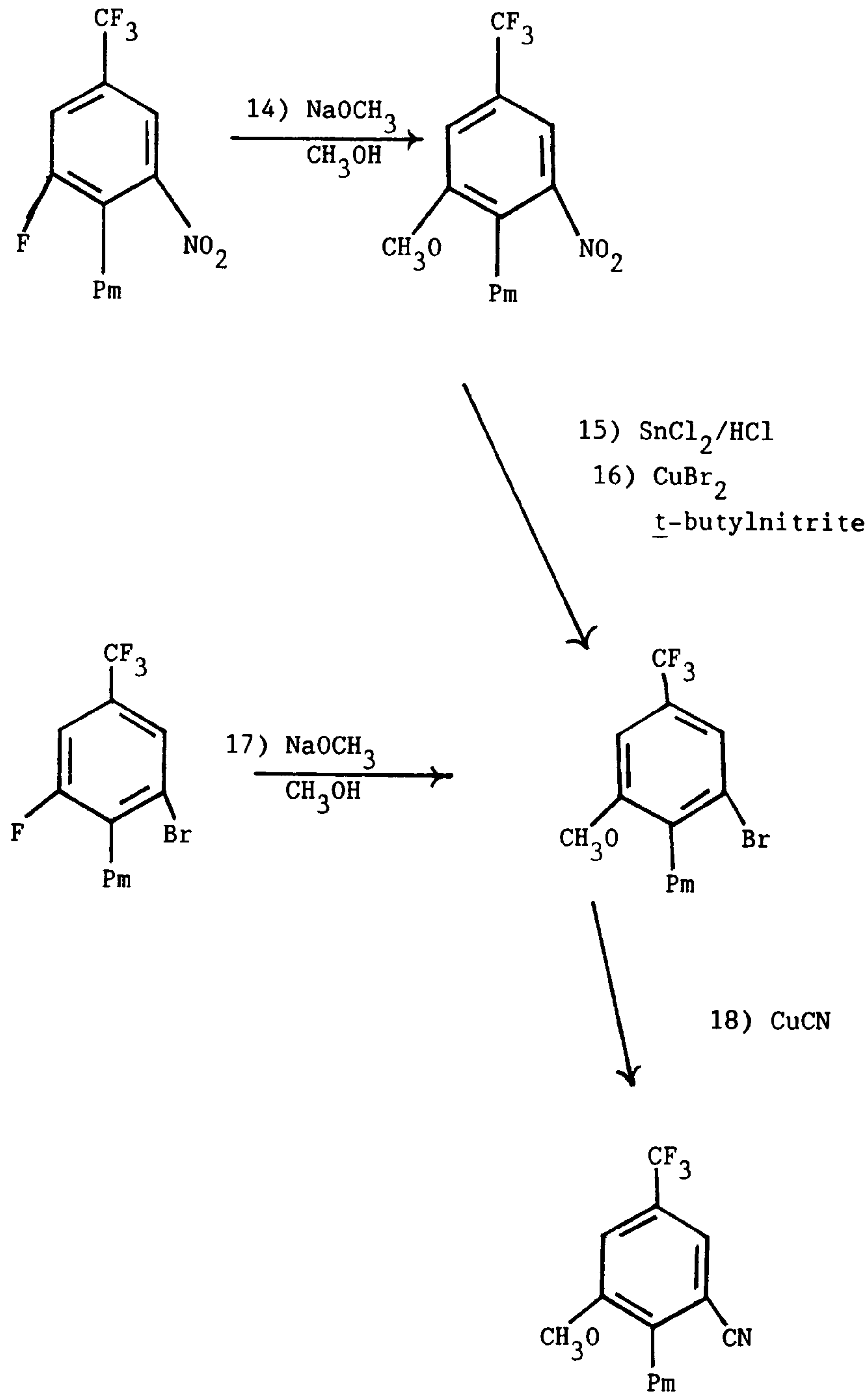
14) NaOCH3
CH3OH
15) SnCl2/HCl
16) CuBr2
t-butylnitrite
17) NaOCH3
CH3OH
18) CuCN

Scheme 8

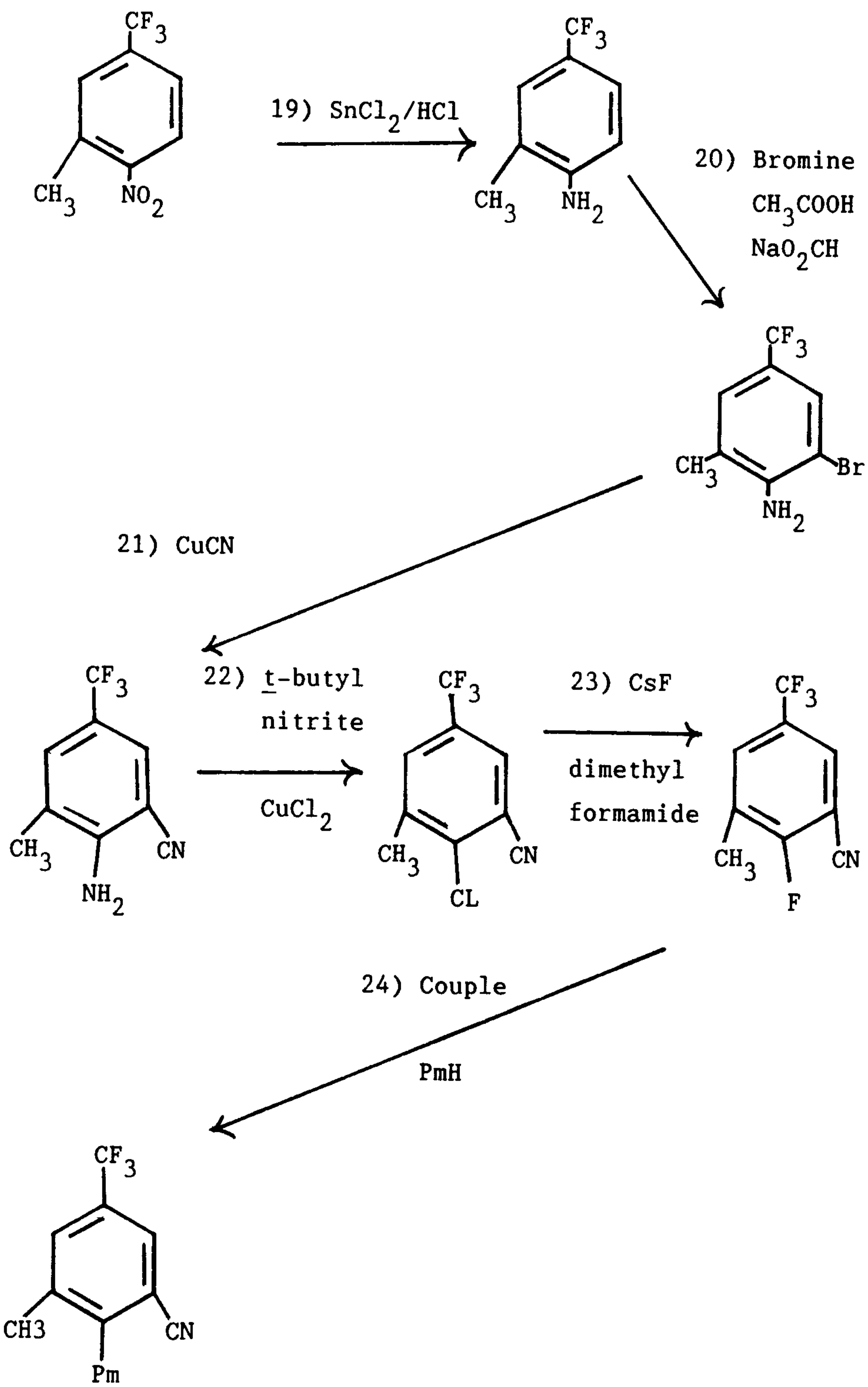


## Claims

1. A compound of formula (I) :

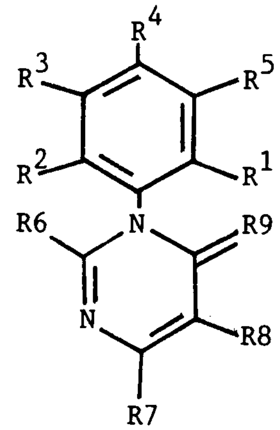

(I)

wherein $R^1$ is cyano or a group $-CX-NR^{10}R^{11}$ wherein X is O or S or S=O; and $R^{10}$ and $R^{11}$ are independently selected from hydrogen, nitro, amino or alkyl optionally substituted by halogen, by cycloalkyl, by formyl, by $C_{2-7}$ alkanoyl, by $C_{4-7}$ cycloalkylcarbonyl, by $C_{2-7}$ alkoxycarbonyl, by $C_{2-7}$ haloalkoxycarbonyl, by an aryl group or by an aromatic heterocyclic group or

$R^{10}$ and $R^{11}$ together with the nitrogen to which they are attached form an aliphatic heterocyclic group containing from 4 to 8 atoms in the ring and optionally substituted by halogen or alkyl or

$R^{10}$ and $R^{11}$ together form the group $=CHR^{12}$ wherein $R^{12}$ is alkyl, $C_{2-6}$ alkenyl, aryl, an aromatic heterocycle, or amino optionally substituted by alkyl or

$R^{10}$ is hydrogen and $R^{11}$ is alkoxycarbonyl, alkylcarbonyl, optionally substituted aralkyl or a group $-S(O)_nR^{13}$ where $R^{13}$ is alkyl, haloalkyl or cycloalkyl and n is 0, 1 or 2;

$R^2$ is hydrogen, halogen, haloalkyl, nitro, cyano, a group $-CX-NR^{10}R^{11}$ as hereinbefore defined, or

$R^2$ is $-C{\equiv}C-Y^1$ wherein $Y^1$ is hydrogen, an optionally halo-substituted alkyl group, an optionally halo-substituted alkoxy group, trialkylsilyl or $-COOY^2$ where $Y^2$ is alkyl, or $Y^1$ is $-CONY^3Y^4$ wherein $Y^3$ and $Y^4$ are independently selected from hydrogen and alkyl,

or $R^2$ is alkyl or alkoxy,

or $R^2$ is $S(O)_nR^{13}$ where $R^{13}$ and n are as previously defined,

or $R^2$ is $NY^5Y^6$ where $Y^5$ and $Y^6$ are independently selected from hydrogen and alkyl;

$R^3$ and $R^5$ are independently selected from hydrogen, halogen, alkyl, haloalkyl, cycloalkyl, nitro, $NY^7Y^6$ wherein $Y^7$ and $Y^6$ are independently selected from hydrogen and alkyl, $-C{\equiv}C-Y^1$, wherein $Y^1$ is as previously defined or $S(O)_nR^{13}$ where $R^{13}$ and n are as previously defined;

$R^4$ is halogen, haloalkyl, haloalkoxy or $S(O)_nR^{13}$ where $R^{13}$ and n are as previously defined;

$R^9$ is oxygen or sulphur;

$R^6$ is hydrogen, halogen, $NR^{14}R^{15}$, $S(O)_nR^{13}$, alkyl or cycloalkyl wherein $R^{14}$ and $R^{15}$ are independently selected from hydrogen, alkyl or cycloalkyl and $R^{13}$ and n are as defined previously;

$R^7$ is halo, nitro, haloalkyl, haloalkoxy or $S(O)_nR^{13}$ where $R^{13}$ and n are as defined previously; and

$R^8$ is $-CR^{16}=CR^{17}R^{18}$ wherein $R^{16}$, $R^{17}$, and $R^{18}$ are independently selected from hydrogen, from alkyl, from halogen, from haloalkyl, from alkoxy, from haloalkoxy, from haloalkylthio, from $COOR^{19}$ where $R^{19}$ is alkyl, from $NR^{20}R^{21}$ where $R^{20}$ and $R^{21}$ are independently selected from hydrogen, alkyl, haloalkyl and cycloalkyl, and from $S(O)_nR^{13}$ where $R^{13}$ and n are as previously defined,

or $R^8$ is $-C{\equiv}C-Y^1$ where $Y^1$ is as previously defined,

or $R^8$ is a group:

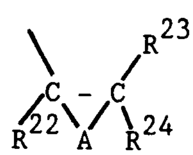

where A is $CH_2$ or CH-halogen or $C(halogen)_2$ or A is O and $R^{22}$, $R^{23}$ and $R^{24}$ are independently selected from hydrogen, alkyl, halogen, or haloalkyl,

or $R^8$ is a group:

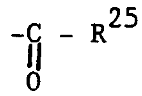

where $R^{25}$ is alkyl or haloalkyl.

**2.** A compound of formula (I'):

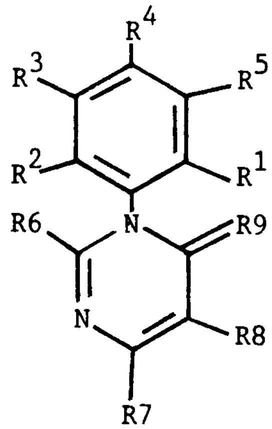

(I')

wherein $R^1$ is cyano or a group $-CX-NR^{10}R^{11}$ wherein X is O or S and $R^{10}$ and $R^{11}$ are independently selected from hydrogen or alkyl;
$R^2$ is hydrogen, halogen, haloalkyl, nitro, cyano or a group $-CX-NR^{10}R^{11}$ as hereinbefore defined;
$R^3$ and $R^5$ are independently selected from hydrogen, halogen, alkyl, haloalkyl, or cycloalkyl;
$R^4$ is halogen, haloalkyl, haloalkoxy or $S(O)_nR^{13}$ where $R^{13}$ and n are as defined in claim 1;
$R^6$ is hydrogen, halogen, $NR^{14}R^{15}$, $S(O)_nR^{13}$, alkyl or cycloalkyl wherein $R^{14}$ and $R^{15}$ are independently selected from hydrogen, alkyl or cycloalkyl and $R^{13}$ and n are as defined previously;
$R^7$ is halo, nitro, haloalkyl, haloalkoxy or $S(O)_nR^{13}$ where $R^{13}$ and n are as defined previously;
$R^8$ is $-CR^{16}=CR^{17}R^{18}$ wherein $R^{16}$, $R^{17}$, and $R^{18}$ are independently selected from hydrogen, from alkyl, from halogen, from haloalkyl, from alkoxy, from haloalkoxy, from haloalkylthio, from $COOR^{19}$ where $R^{19}$ is alkyl, from $NR^{20}R^{21}$ where $R^{20}$ and $R^{21}$ are independently selected from hydrogen, alkyl, haloalkyl and cycloalkyl, and from $S(O)_nR^{13}$ where $R^{13}$ and n are as previously defined,
or $R^8$ is $-C\equiv C-Y^1$ where $Y^1$ is hydrogen, alkyl or haloalkyl,
or $R^8$ is a group:

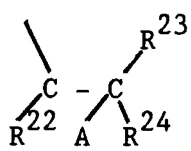

where A is $CH_2$ or CH(halogen) or $C(halogen)^2$ or A is O and $R^{22}$, $R^{23}$ and $R^{24}$ are independently selected from hydrogen, alkyl, halogen, or haloalkyl,
or $R^8$ is a group:

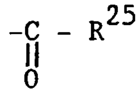

where $R^{25}$ is alkyl or haloalkyl; and
$R^9$ is oxygen or sulphur.

3. A compound according to claim 1 or claim 2 wherein $R^4$ is trifluoromethyl, pentafluoroethyl trifluoromethylthio, iodine, bromine, chlorine, trifluoromethoxy or methylthio or $-SOCF_3$.

4. A compound according to any of the preceding claims wherein $R^3$, $R^5$ and $R^6$ are all hydrogen.

5. A compound according to any of the preceding claims wherein $R^2$ is fluorine, chlorine, bromine, cyano, or trifluoromethyl.

6. A compound according to any of the preceding claims wherein $R^1$ is cyano.

7. A compound according to any of the preceding claims wherein $R^7$ is trifluoromethyl or pentafluoroethyl.

8. A compound according to any of the preceding claims wherein when $R^8$ is $-CR^{16}=CR^{17}R^{18}$ then $R^{16}$, $R^{17}$ and $R^{18}$ are independently selected from hydrogen, methyl, ethyl, methoxy and ethoxy; or when $R^8$ is $-C{\equiv}C-Y^1$ then $Y^1$ is hydrogen or alkyl; or when $R^8$ is a group

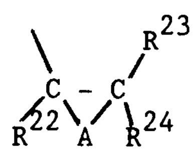

then $R^{22}$, $R^{23}$, and $R^{24}$ are independently selected from hydrogen, methyl or ethyl; or when $R^8$ is a group

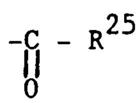

then $R^{25}$ is methyl or ethyl.

9. A compound according to any of the preceding claims wherein $R^9$ is oxygen.

10. A method of preparing a compounds of formula (I) as defined in claim 1 or claim 2 which comprises reacting a compound of formula (II) :

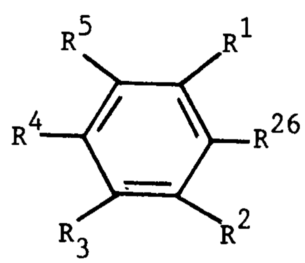

(II)

wherein $R^1$, $R^2$, $R^3$, $R^4$ and $R^5$ are as defined in relation to formula (I) and $R^{26}$ is a leaving group with a compound of formula (III) :

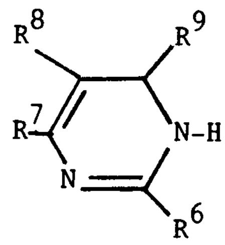

(III)

wherein $R^6$, $R^7$, $R^8$ and $R^9$ are as defined in relation to formula (I) and thereafter if desired (a) converting a group $R^2$-$R^5$ to a different such group, and/or (b) converting a group $R^6$ to $R^9$ to a different such group.

**11.** A method of killing or controlling insect or acarine pests which method comprises applying to the pest or to a locus thereof an insecticidally or acaricidally effective amount of a compound of formula (I) as defined in claim 1 or claim 2.

**12.** An insecticidal or acaricidal composition comprising a compound of formula (I) as defined in claim 1 or claim 2 in combination with a diluent or carrier.

European Patent Office

# EUROPEAN SEARCH REPORT

Application Number

EP 91308508.0

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP - A - 0 338 686 (ICI) * Claims 1,8,9 * -- | 1,11, 12 | C 07 D 239/36<br>A 01 N 43/54 |
| P,A | EP - A - 0 396 250 (ICI) * Claims 1,14,15,16,17, 29,30 * -- | 1,10, 11,12 | |
| A | GB - A - 1 447 108 (SHELL) * Claim 1 * ---- | 1 | |
| | | | **TECHNICAL FIELDS SEARCHED (Int. Cl.5)** |
| | | | A 01 N 43/00<br>C 07 D 239/00 |

The present search report has been drawn up for all claims

| Place of search | Date of completion of the search | Examiner |
|---|---|---|
| VIENNA | 27-11-1991 | LUX |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)